# EUROPEAN PATENT APPLICATION

(11) **EP 1 806 585 A1**
(43) Date of publication of application: **11.07.2007**
(21) Application number: 07100346.1
(22) Date of filing: 10.03.2004
(51) Int. Cl.: G01N 33/574, C07H 21/00, A61K 31/70

(54) **Detection, monitoring and treatment of cancer**

(30) Priority: 10.03.2003 GB 0305422
(62) Divisional of application: 04719000.4
(71) Applicant: THE OPEN UNIVERSITY, Milton Keynes MK7 6AA (GB)
(72) Inventor: Bruce, James Ironside, Milton Keynes, MK7 6AA (GB); Missailidis, Sotiris, Milton Keynes, Buckinghamshire MK7 6AA (GB); Ferreira, Catia Sofia Matos Room 7-105 610, Toronto Ontario M5G 2M9 (CA); Borbas, Katalin Eszter, Raleigh, NC NC 27695-8204 (US)
(74) Representative: Eddowes, Simon

(57) **Abstract**

There are disclosed compounds having the structure: 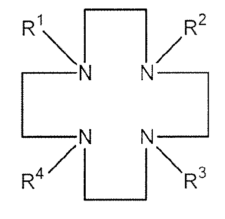 wherein R¹, R², R³ are each independently:
hydrogen, R⁴, R⁵, R⁵-carbonyl, R⁵-sulphonyl;
R⁴ comprises an amino acid moiety;
R⁵ is:
alkyl, alkenyl, alkynyl, cycloalkyl, aryl, heteroaryl, heterocyclyl, wherein each of these groups is optionally substituted by one or more of:
alkyl, amino, amino-carbonyl, amino-sulphonyl, halo, cyano, hydroxy, nitro, trifluoromethyl, alkoxy, alkoxycarbonyl, aryl and alkylthio;
and salts and solvates thereof.

## Description

This invention relates to compounds which are useful in the detection, monitoring and treatment of cancer and to the detection, monitoring and treatment of cancer using such compounds.

Cancer is a major cause of morbidity in the world. In the UK specifically, nearly 255,000 new cases (excluding non-melanoma skin cancer) were registered in 1996. Translated into individual risk, this means that over 1 in 3 people are at risk of developing cancer during their lifetime. Cancer is also the cause of a quarter of all deaths in the UK. In 1998 there were 155,000 deaths from cancer - nearly a quarter of these were from lung cancer and a further quarter were caused by cancers of the large bowel, breast and prostate. Breast cancer is by far the commonest cancer for women, with around 35,000 new cases in the UK alone in 1996. Large bowel is the second most common cancer in women, followed by lung and ovary cancer. The commonest cancer for men is lung cancer, responsible for a fifth of all new cases. Prostate cancer is the second most common cancer in men, followed by cancer of the large bowel and bladder cancer.

For many years work has been carried out on the identification of molecules characteristic and unique in tumours, or molecules altered, overexpressed or otherwise differing in cancer. These molecules are known as tumour markers and have been used over the years as the target for many immunological approaches to cancer diagnosis and therapy.

MUC1 epithelial mucin is such a molecule. This epithelial mucin is coded for by the MUC1 gene. It is not a classic extracellular complex mucin, such as those found as major components of the mucous layers covering the gastrointestinal and respiratory tracts, but is a transmembrane molecule, expressed by most glandular epithelial cells. The protein consists of a number of distinct regions, including an N-terminus with a putative signal peptide and degenerate tandem repeats, a transmembrane region and a C-terminus cytoplasmic tail. The major portion of the protein is the tandem repeat region. This consists of degenerate repeats of the unique peptide sequence APDTRPAPGSTAPFAHGVTS. The number of repeats varies with the allele, thus making the gene and protein highly polymorphic. MUC1 is also referred to as MUC-1, mucin 1, muc-1, episialin, peanut-reactive urinary mucin (PUM), polymorphic epithelial mucin (PEM), CD227, epithelial membrane antigen (EMA), DF3 antigen and H23 antigen.

MUC1 mucin is restricted to the apical cell surface by interactions with the microfilament network. Although MUC1 is widely expressed by normal glandular epithelial cells, the expression is dramatically increased when the cells become malignant. This has been well documented for breast and ovarian cancer, as well as some lung, pancreatic and prostate cancers. Recently it has also been shown that MUC1 is a valuable marker for bladder cancer and has been used for its diagnosis in a number of studies. Antibody studies have also shown not only that MUC1 is overexpressed in carcinomas but also that the pattern of glycosylation is altered. Thus, in the breast cancer mucin, glycosylation changes result in certain epitopes in the core protein being exposed which are masked in the mucin produced by the lactating mammary gland. These characteristics of MUC1 have been explored over the years in a number of immunotherapeutic approaches, mainly involving radiolabelled antibodies against breast and bladder cancers. Other attempts on active specific immunotherapies based on MUC1 have also taken place in animal models to investigate the efficacy of immunogens based on MUC1.

These immunotherapeutic approaches had some encouraging results and have led to clinical trials both for the vaccine therapies and the antibody treatments. These strategies however are not without problems. The radiolabelled antibody technique is limited to modest (millicurie) radiation doses since the long circulation time of radiolabelled antibodies makes bone marrow toxicity a problem. Another problem is the time period required to produce specific monoclonal antibodies. Additionally, recent attempts to use peptides instead of antibodies have resulted in molecules with very low affinity for the MUC1 mucin.

The present invention seeks to overcome or alleviate at least some of these problems.

According to the present invention, there is provided an aptamer ligand to MUC1. Preferably the aptamer is a DNA/RNA oligonucleotide comprising natural, modified and/or unnatural nucleotides.

According to another aspect of the present invention, there is provided an aptamer comprising a sequence selected from:
1 CGAATGGGCCCGTCCTCGCTGTAAG
2 GCAACAGGGTATCCAAAGGATCAAA
3 GTTCGACAGGAGGCTCACAACAGGC
4 TGTTGGTCAGGCGGCGGCTCTACAT
5 CTCTGTTCTTATTTGCGAGTTXXXX
6 XCTCTGTTCTTATTTGCGAGTTXXX
7 XXCTCTGTTCTTATTTGCGAGTTXX
8 XXXCTCTGTTCTTATTTGCGAGTTX
9 XXXXCTCTGTTCTTATTTGCGAGTT
10 CTCTGTTCTTATTTGCGAGTT
11 CCCTCTGTTCTTATTTGCGAGTTCA
12 CTCTGTTCTTATTTGCGAGTTGGTG
13 CCCTCTGTTCTTATTTGCGAGTTCA
14 TAAGAACAGGGCGTCGTGTTACGAG
15 GTGGCTTACTGCGAGGACGGGCCCA
16 GCAGTTGATCCTTTGGATACCCTGG
17 AACCCTATCCACTTTTCGGCTCGGG
18 CGATTTAGTCTCTGTCTCTAGGGGT
19 CGACAGGAGGCTCACAACAGGCAAC
20 AGAACGAAGCGTTCGACAGGAGGCT
21 AGAAACACTTGGTATATCGCAGATA
22 GGGAGACAAGAATAAACACTCAACG
wherein each X is independently a natural, non-natural, modified or derivatised nucleic acid.

Preferably, each X is independently selected from G, C, A, T and U.

According to a further aspect of the present invention, there is provided an aptamer comprising a sequence substantially homologous to a sequence defined above.

Preferably, the aptamer comprises a sequence which is at least 90% homologous to a sequence defined above.

Conveniently the aptamer comprises a sequence which is at least 80% homologous to a sequence defined above.

Advantageously, the aptamer comprises a sequence which is at least 70% homologous to a sequence defined above.

According to yet another aspect of the present invention, there is provided an aptamer ligand to MUC1 comprising a tertiary structure substantially the same as the tertiary structure of an aptamer as defined above.

According to a yet further aspect of the present invention, there is provided an aptamer which has a mode of binding to MUC1 which is substantially the same as that of an aptamer as defined above.

Preferably, the aptamer comprises a modified and/or non-natural nucleotide.

Conveniently, the aptamer comprises a modified sugar group.

Advantageously, the modified sugar group has an amino group.

Preferably, the modified or non-natural nucleotide or group is at the 3' end of the aptamer.

According to another aspect of the present invention, there is provided a compound having the structure: 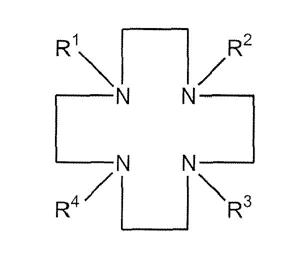 wherein
R¹, R², R³ are each independently:
   hydrogen, R⁴, R⁵, R⁵-carbonyl, R⁵-sulphonyl;
R⁴ comprises an amino acid moiety;
R⁵ is:
   alkyl, alkenyl, alkynyl, cycloalkyl, aryl, heteroaryl, heterocyclyl, wherein each of these groups is optionally substituted by one or more of:
   alkyl, amino, amino-carbonyl, amino-sulphonyl, halo, cyano, hydroxy, nitro, trifluoromethyl, alkoxy, alkoxycarbonyl, aryl and alkylthio;
and salts and solvates thereof.

Preferably, R⁴ comprises a lysine, cysteine, glycine, threonine, serine, arginine or methionine moiety.

Conveniently R¹, R² and R³ are hydrogen.

Advantageously, at least one of R¹, R² and R³ is alkyl.

Preferably, R⁴ comprises a carboxyl or amino group.

Conveniently, R⁴ comprises a nitrogen, oxygen or sulphur atom.

Advantageously, R⁴ has the structure:

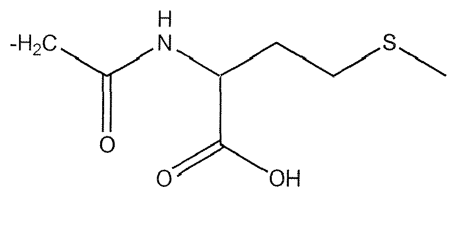

Preferably, the compound has the structure:

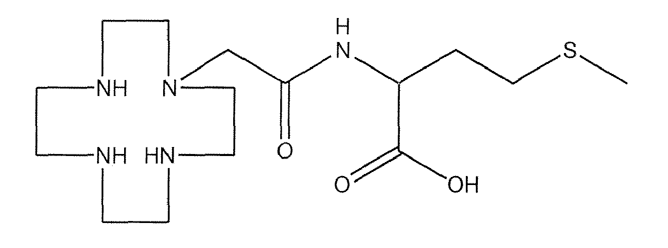

Conveniently, the compound further contains a complexing metal (M).

According to another aspect of the present invention, there is provided a compound having the structure: 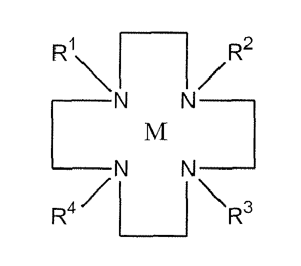 wherein
R¹, R², R³ are each independently:
   hydrogen, R⁴, R⁵, R⁵-carbonyl, R⁵-sulphonyl;
R⁴ comprises an amino acid moiety;
R⁵ is:
   alkyl, alkenyl, alkynyl, cycloalkyl, aryl, heteroaryl, heterocyclyl, wherein each of these groups is optionally substituted by one or more of:
   alkyl, amino, amino-carbonyl, amino-sulphonyl, halo, cyano, hydroxy, nitro, trifluoromethyl, alkoxy, alkoxycarbonyl, aryl and alkylthio;
   and salts and solvates thereof.

Preferably, R⁴ comprises a lysine, cysteine, glycine, threonine, serine, arginine or methionine moiety.

Conveniently, R¹, R² and R³ are hydrogen.

Advantageously, at least one of R¹, R² and R³ is alkyl.

Preferably, R⁴ comprises a carboxyl or amino group.

Conveniently, R⁴ comprises a nitrogen, oxygen or sulphur atom.

Advantageously, R⁴ has the structure:

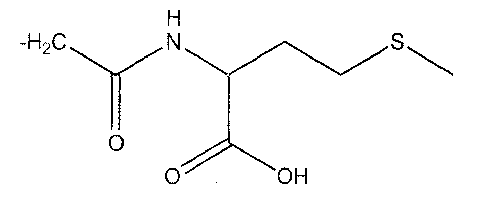

Preferably, the compound has the structure:

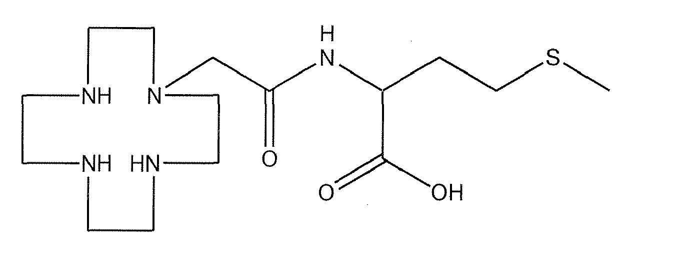

Conveniently, M is rhenium, technitium or yttrium.

Advantageously, M is a radioisotope.

According to a further aspect of the present invention, there is provided a compound comprising an aptamer as defined above and a non-nucleic acid moeity.

Preferably, the non-nucleic acid moeity comprises a ligand for a metal.

Conveniently, the non-nucleic acid moeity comprises a nitrogen-containing ring.

Advantageously, the non-nucleic acid moeity comprises a cyclen.

Preferably, the non-nucleic acid moeity comprises a compound as defined above.

Conveniently, the non-nucleic acid moeity comprises a porphyrin.

Advantageously, the non-nucleic acid moeity comprises the compound having the formula:

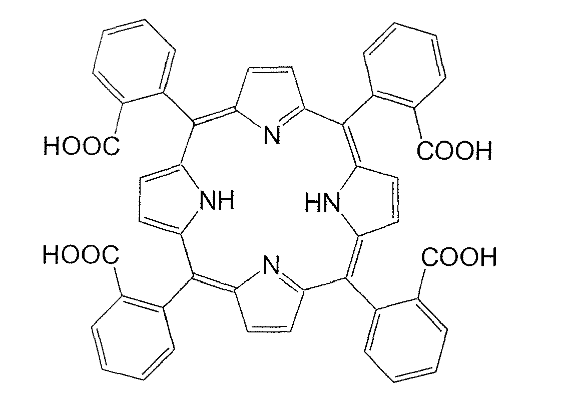

Preferably, the non-nucleic acid moeity comprises a sulphur-containing group.

Advantageously, the non-nucleic acid moeity comprises the moeity:

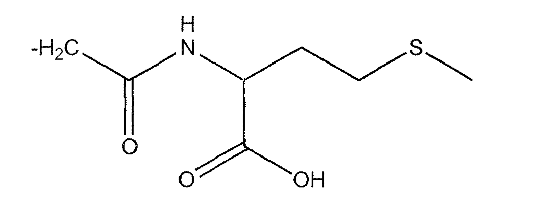

Preferably, an amino group of the aptamer is connected to a carboxyl group of the non-nucleic acid moeity.

Conveniently, the amino group of the aptamer is located on a sugar.

Advantageously, the aptamer is connected to the non-nucleic acid moeity by a spacer.

Preferably, the spacer is selected from phosphoramidite 9, phosphoramidite C3, dSpacer CE phosphoramidite, spacer phosphoramidite 18, spacer C12 CE phosphoramidite, 3' spacer CE.

Conveniently, the spacer is a peptide moeity.

Preferably, the non-nucleic acid moeity has therapeutic activity.

Conveniently, the non-nucleic acid moeity has diagnostic activity.

Advantageously, the non-nucleic acid moeity is remotely detectable.

According to a further aspect of the present invention, there is provided a compound comprising an aptamer as defined above, a non-nucleic acid moeity and a complexing metal (M).

Preferably, the non-nucleic acid moeity comprises a ligand for a metal.

Conveniently, the non-nucleic acid moeity comprises a nitrogen-containing ring.

Advantageously, the non-nucleic acid moeity comprises a cyclen.

Preferably, the non-nucleic acid moeity comprises a compound as defined above.

Conveniently, the non-nucleic acid moeity comprises a porphyrin.

Advantageously, the non-nucleic acid moeity comprises the compound having the structure: 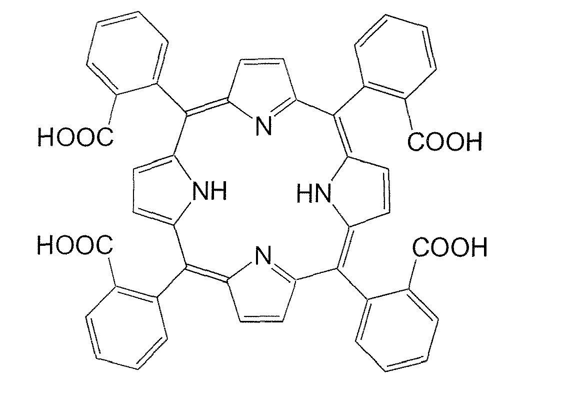 Preferably, the non-nucleic acid moeity comprises a sulphur-containing group.

Conveniently, the non-nucleic acid moeity comprises the moeity:

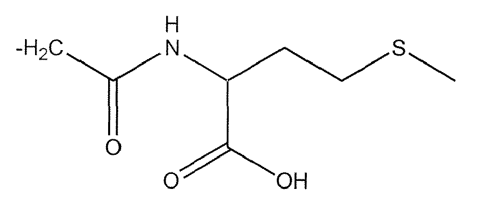

Advantageously, an amino group of the aptamer is connected to a carboxyl group of the non-nucleic acid moeity.

Preferably, the amino group of the aptamer is located on a sugar.

Conveniently, the aptamer is connected to the non-nucleic acid moeity by a spacer.

Advantageously, the spacer is selected from phosphoramidite 9, phosphoramidite C3, dSpacer CE phosphoramidite, spacer phosphoramidite 18, spacer C12 CE phosphoramidite, 3' spacer CE.

Preferably, the spacer is a peptide moeity.

Advantageously, M is rhenium, technitium or yttrium.

Preferably, M is a radioisotope.

Conveniently, the non-nucleic acid moeity has therapeutic activity.

Advantageously, the non-nucleic acid moeity has diagnostic activity.

Preferably, the non-nucleic acid moeity is remotely detectable.

Advantageously, the non-nucleic acid moiety has imaging properties.

According to yet another aspect of the present invention, there is provided a pharmaceutical composition comprising a compound or aptamer as defined above and a pharmaceutically acceptable carrier.

According to a yet further aspect of the invention, there is provided a method of preventing and/or treating a disease or condition comprising the step of administering a compound, aptamer or pharmaceutical composition as defined in ary of the preceding claims to a subject.

Preferably, the disease or condition is associated with the over-production of MUC1.

Conveniently, the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

Advantageously, MUC1 bearing and/or expressing cells are irradiated by the compound, aptamer or pharmaceutical composition.

According to another aspect of the invention, there is provided a method of diagnosing a disease or condition comprising the step of administering a compound, aptamer or pharmaceutical composition as defined above to a sample or a subject.

Preferably, the disease or condition is associated with the over-production of MUC1.

Conveniently, the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

Advantageously, MUC1 bearing and/or expressing cells are irradiated by the compound, aptamer or pharmaceutical composition.

According to a further aspect of the invention, there is provided a method of detecting a disease or condition comprising the step of administering a compound, aptamer or pharmaceutical composition as defined above to a sample or subject.

Preferably, the disease or condition is associated with the over-production of MUC1.

Conveniently, the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

Advantageously, MUC1 bearing and/or expressing cells are irradiated by the compound, aptamer or pharmaceutical composition.

According to another aspect of the present invention, there is provided a method of imaging a disease or condition comprising the step of administering a compound, aptamer or pharmaceutical composition as defined above to a sample or subject.

Preferably, the disease or condition is associated with the over-production of MUC1.

Conveniently, the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

Advantageously, MUC1 bearing and/or expressing cells are irradiated by the compound, aptamer or composition.

According to a yet further aspect of the present invention, there is provided a compound, aptamer or composition of the invention for use in therapy.

Preferably, the compound, aptamer or composition of the invention for use in the treatment and/or prevention of a condition or disease associated with the over-production of MUC1.

Conveniently, the compound or aptamer of the invention is for use in the treatment and/or prevention of a disease or condition selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to yet another aspect of the invention, there is provided a compound, aptamer or composition of the invention for use in diagnosis.

Preferably, the compound, aptamer or composition of the invention is for use in the diagnosis of a condition or disease associated with the over-production of MUC1.

Conveniently, the compound, aptamer or composition of the invention is for use in the diagnosis of a disease or condition selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to a further aspect of the invention, there is provided a compound, aptamer or composition of the invention for use in the detection of a disease or condition.

Preferably, the condition or disease is associated with the over-production of MUC1.

Conveniently, the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to another aspect of the invention, there is provided a compound, aptamer or composition of the invention for use in the imaging of a disease or condition.

Preferably, the condition or disease is associated with the over-production of NIUC1.

Conveniently, wherein the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to a further aspect of the invention, there is provided the use of a compound or aptamer as defined above for the manufacture of a medicament for the prevention and/or treatment of a disease or condition associated with the over-production of MUC1.

According to yet another aspect of the invention, there is provided the use of a compound or aptamer as defined above for the manufacture of a medicament for the prevention and/or treatment of a disease or condition selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to another aspect of the invention, there is provided the use of a compound or aptamer as defined above for the manufacture of a product for the diagnosis of a disease or condition associated with the over-production of MUC1.

According to a further aspect of the invention, there is provided the use of a compound or aptamer as defined above for the manufacture of a product for the diagnosis of a disease or condition selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to yet another aspect of the invention, there is provided the use of a compound or aptamer as defined above for the manufacture of a product for the detection of a disease or condition associated with the over-production of MUC1.

According to a further aspect of the invention, there is provided the use of a compound or aptamer as defined above for the manufacture of a product for the detection of a disease or condition selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to another aspect of the invention, there is provided the use of a compound or aptamer as defined above for the manufacture of a product for the imaging of a disease or condition associated with the over-production of MUC1.

According to a yet further aspect of the invention, there is provided the use of a compound or aptamer as defined above for the manufacture of a product for the imaging of a disease or condition selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

According to yet another aspect of the invention, there is provided a kit for the prevention, treatment, diagnosis, detection and/or imaging of a disease or condition comprising a compound, aptamer or composition of the invention.

According to a further aspect of the invention, there is provided a kit for the prevention, treatment, diagnosis, detection and/or imaging of a disease or condition associated with the over-production of MUC1 comprising a compound, aptamer or composition of the invention.

According to another aspect of the invention, there is provided a kit for the prevention, treatment, diagnosis, detection and/or imaging of a disease or condition selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer, comprising a compound, aptamer or composition of the invention.

According to a yet further aspect of the invention, there is provided a method of preparing a compound having the structure: 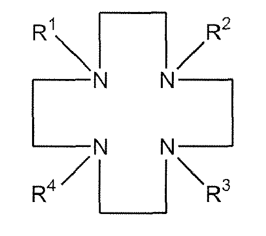 wherein
R¹, R², R³ are each independently:
   hydrogen, R⁴, R⁵, R⁵-carbonyl, R⁵-sulphonyl;
R⁴ comprises an amino acid moiety;
R⁵ is:
   alkyl, alkenyl, alkynyl, cycloalkyl, aryl, heteroaryl, heterocyclyl, wherein each of these groups is optionally substituted by one or more of:
   alkyl, amino, amino-carbonyl, amino-sulphonyl, halo, cyano, hydroxy, nitro, trifluoromethyl, alkoxy, alkoxycarbonyl, aryl and alkylthio;
and salts and solvates thereof;
comprising the step of reacting a cyclen compound with an amino acid moiety.

Preferably, R⁴ comprises a lysine, cysteine, glycine, threonine, serine, arginine or methionine moiety.

Conveniently, R¹, R² and R³ are hydrogen.

Advantageously, at least one of R¹, R² and R³ is alkyl.

Preferably, R⁴ comprises a carboxyl or amino group.

Conveniently, R⁴ comprises a nitrogen, oxygen or sulphur atom.

Accordingly, R⁴ has the structure:

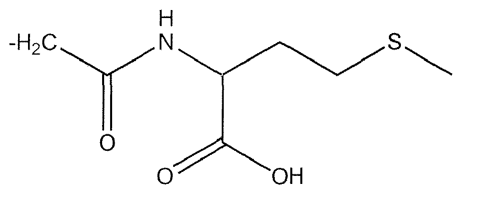

Preferably, the compound has the structure:

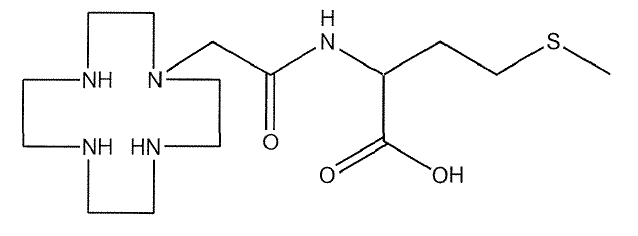

Conveniently, the method has the following synthetic route:

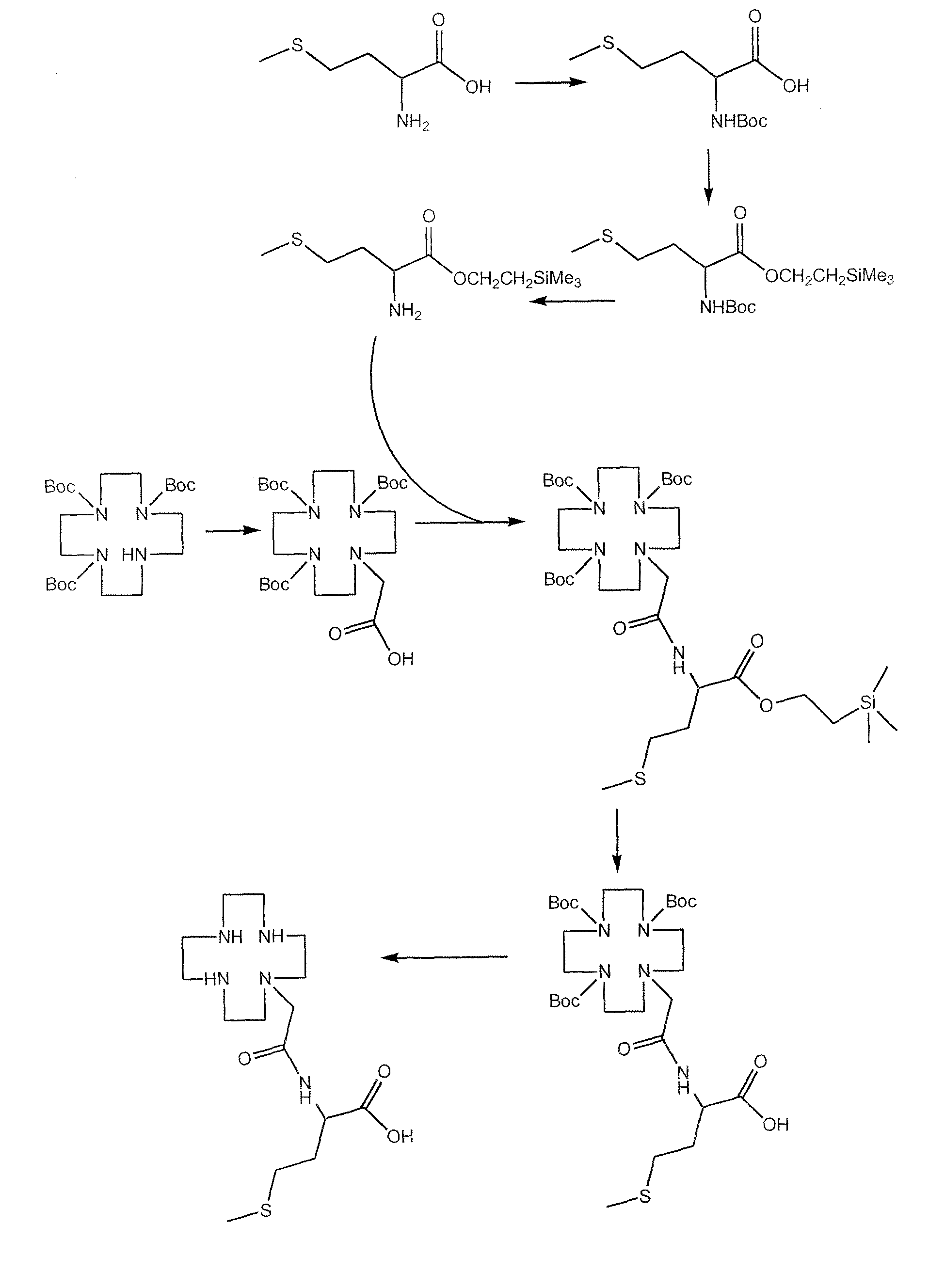

Advantageously, the method has the following synthetic route:

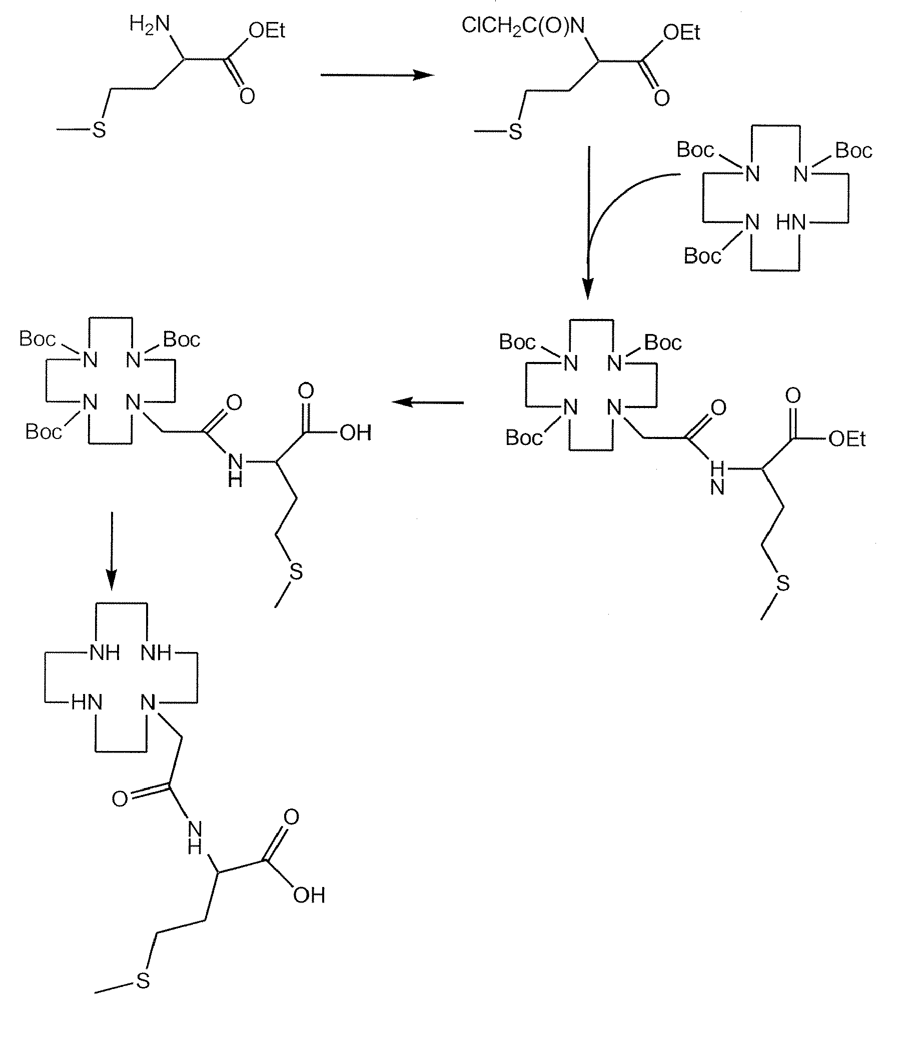

According to yet another aspect of the present invention, there is provided a method of making an aptamer comprising a sequence selected from:
1 CGAATGGGCCCGTCCTCGCTGTAAG
2 GCAACAGGGTATCCAAAGGATCAAA
3 GTTCGACAGGAGGCTCACAACAGGC
4 TGTTGGTCAGGCGGCGGCTCTACAT
5 CTCTGTTCTTATTTGCGAGTTXXXX
6 XCTCTGTTCTTATTTGCGAGTTXXX
7 XXCTCTGTTCTTATTTGCGAGTTXX
8 XXXCTCTGTTCTTATTTGCGAGTTX
9 XXXXCTCTGTTCTTATTTGCGAGTT
10 CTCTGTTCTTATTTGCGAGTT
11 CCCTCTGTTCTTATTTGCGAGTTCA
12 CTCTGTTCTTATTTGCGAGTTGGTG
13 CCCTCTGTTCTTATTTGCGAGTTCA
14 TAAGAACAGGGCGTCGTGTTACGAG
15 GTGGCTTACTGCGAGGACGGGCCCA
16 GCAGTTGATCCTTTGGATACCCTGG
17 AACCCTATCCACTTTTCGGCTCGGG
18 CGATTTAGTCTCTGTCTCTAGGGGT
19 CGACAGGAGGCTCACAACAGGCAAC
20 AGAACGAAGCGTTCGACAGGAGGCT
21 AGAAACACTTGGTATATCGCAGATA
22 GGGAGACAAGAATAAACACTCAACG
wherein each X is independently a natural, non-natural, modified or derivatised nucleic acid;
comprising the step of reacting nucleic acid compounds together.

Preferably, each X is independently selected from the group of nucleic acids consisting of G, C, A, T and U.

According to a further aspect of the invention, there is provided a method of making an aptamer comprising a sequence substantially homologous to a sequence defined above comprising the step of reacting nucleic acid compounds together.

Preferably, the aptamer comprises a sequence which is at least 90% homologous to a sequence defined above.

Conveniently, the aptamer comprises a sequence which is at least 80% homologous to a sequence defined above.

Advantageously, the aptamer comprises a sequence which is at least 70% homologous to a sequence defined above.

According to another aspect of the invention, there is provided a method of making an aptamer comprising a tertiary structure substantially the same as an aptamer of the invention comprising the step of reacting nucleic acid compounds together.

Preferably, the nucleic acid compounds comprise modified and/or unnatural amino acid compounds.

Conveniently, the aptamer comprises a modified sugar group.

Advantageously, the modified sugar group has an amino group.

Preferably, the modified or non-natural group or nucleotide is at the 3' end of the aptamer.

According to a yet further aspect of the invention, there is provided a method of preparing a compound comprising a non-nucleic acid moeity and an aptamer comprising a sequence selected from:
1 CGAATGGGCCCGTCCTCGCTGTAAG
2 GCAACAGGGTATCCAAAGGATCAAA
3 GTTCGACAGGAGGCTCACAACAGGC
4 TGTTGGTCAGGCGGCGGCTCTACAT
5 CTCTGTTCTTATTTGCGAGTTXXXX
6 XCTCTGTTCTTATTTGCGAGTTXXX
7 XXCTCTGTTCTTATTTGCGAGTTXX
8 XXXCTCTGTTCTTATTTGCGAGTTX
9 XXXXCTCTGTTCTTATTTGCGAGTT
10 CTCTGTTCTTATTTGCGAGTT
11 CCCTCTGTTCTTATTTGCGAGTTCA
12 CTCTGTTCTTATTTGCGAGTTGGTG
13 CCCTCTGTTCTTATTTGCGAGTTCA
14 TAAGAACAGGGCGTCGTGTTACGAG
15 GTGGCTTACTGCGAGGACGGGCCCA
16 GCAGTTGATCCTTTGGATACCCTGG
17 AACCCTATCCACTTTTCGGCTCGGG
18 CGATTTAGTCTCTGTCTCTAGGGGT
19 CGACAGGAGGCTCACAACAGGCAAC
20 AGAACGAAGCGTTCGACAGGAGGCT
21 AGAAACACTTGGTATATCGCAGATA
22 GGGAGACAAGAATAAACACTCAACG
wherein each X is independently a natural, non-natural, modified or derivatised nucleic acid;
comprising the step of reacting the aptamer with a non-nucleic acid moiety.

Preferably, each X is independently selected from G, C, A, T and U.

Conveniently, the aptamer comprises modified and/or non-natural nucleotides.

Advantageously, the aptamer comprises a modified sugar group.

Preferably, the modified sugar group has an amino group.

Conveniently, the modified or non-natural group or nucleotide is at the 3' end of the aptamer.

Advantageously, the non-nucleic acid moeity comprises a ligand for a metal.

Preferably, the non-nucleic acid moeity comprises a nitroaen-containing ring.

Conveniently, the non-nucleic acid moeity comprises a cyclen.

Advantageously, the non-nucleic acid moeity comprises a compound as defined above.

Preferably, the non-nucleic acid moeity comprises a porphyrin.

Conveniently, the non-nucleic acid moeity comprises the compound having the structure: 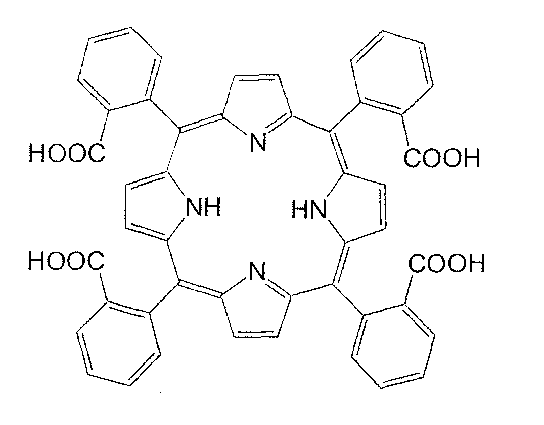 Advantageously, the non-nucleic acid moeity comprises a compound as defined above.

Preferably, the aptamer and the non-nucleic acid moeity are reacted under peptide coupling conditions.

Conveniently, the method comprises the step of reacting an amino moiety with a carbonylic acid moiety.

Advantageously, the method comprises the step of coupling a carboxyl group of the non-nucleic acid moeity with an amino group of the aptamer.

According to yet another aspect of the invention, there is provided a method of complexing a compound with a complexing metal (M) comprising the step of admixing a compound having the structure: 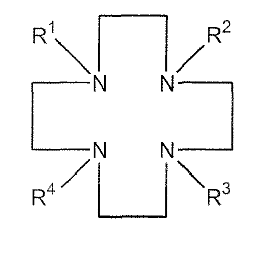 wherein
R¹, R², R³ are each independently:
   hydrogen, R⁴, R⁵, R⁵-carbonyl, R⁵-sulphonyl;
R⁴ comprises an amino acid moiety;
R⁵ is:
   alkyl, alkenyl, alkynyl, cycloalkyl, aryl, heteroaryl, heterocyclyl, wherein each of these groups is optionally substituted by one or more of:
   alkyl, amino, amino-carbonyl, amino-sulphonyl, halo, cyano, hydroxy, nitro, trifluoromethyl, alkoxy, alkoxycarbonyl, aryl and alkylthio;
   and salts and solvates thereof;
   with a source of M.

Preferably, R⁴ comprises a lysine, cysteine, glycine, threonine, serine, arginine or methionine moiety.

Conveniently, R¹, R² and R³ are hydrogen.

Advantageously, at least one of R¹, R² and R³ is alkyl.

Preferably, R⁴ comprises a carboxyl or amino group.

Conveniently, R⁴ comprises a nitrogen, oxygen or sulphur atom.

Advantageously, R⁴ has the structure:

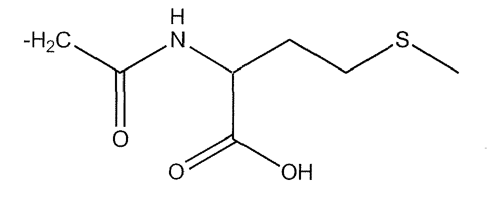

Preferably, the compound has the structure:

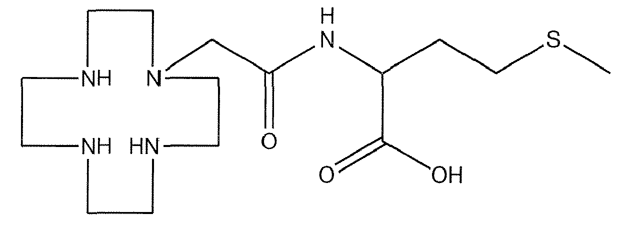

Conveniently, the method comprises the step of reacting the compound with NaMO₄ /NaBH₄.

Advantageously, M is selected from rhenium, technitium and yttrium.

Preferably, M is a radioisotope.

According to a further aspect of the invention, there is provided a method of identifying an aptamer ligand to a target comprising the steps of:
I, attaching a target to a solid support,
ii, admixing a library of aptamers with the attached target,
iii, eluting any non-binding aptamers from the solid support,
iv, releasing any binding aptamers from the attached target,
v, eluting any binding aptamers from the solid support,
vi, amplifying any binding aptamers,
vii, identifying any binding aptamers.

Preferably, the solid support comprises sepharose, agarose, cellulose, activated resin, modified/activated beads, magnetic beads, glass beads or plastic beads.

Conveniently, the target is attached to the solid support by the reaction of a amino group of the target with the solid support.

Advantageously, the amplification step comprises the use of PCR.

Preferably, the amplification step comprises the use of unidirectional PCR.

Conveniently, the amplification step comprises the step of reverse transcription.

Advantageously, the identification step comprises the step of sequencing the aptamer.

Preferably, the method further comprises the step of repeating the steps ii, to vi, with the amplified aptamers.

Conveniently, the steps ii, to vi, are repeated for at least 2 cycles.

Advantageously, the steps ii, to vi, are repeated for at least 3 cycles.

Preferably, the steps ii, to vi, are repeated for at least 5 cycles.

Conveniently, the steps ii, to vi, are repeated for at least 10 cycles.

Advantageously, the target is a peptide.

Preferably, the target is a fragment of a larger target.

Conveniently, the target comprises at least a part of MUC1.

Advantageously, the library of aptamers comprises a 25 nucleic acid variable region.

Conveniently, the aptamers further comprise primers.

Advantageously, the primers are selected from:
GGGAGACAAGAATAAACGCTCAA, and
TTCGACAGGAGGCTCACAACAGGC.

In this specification, the following terms have the following definitions:
"Alkyl" means a straight-chain or branched-chain alkyl group containing preferably 1 to 16 carbon atoms, more preferably 1 to 8 carbon atoms, still more preferably 1 to 4 carbon atoms, e.g. methyl, ethyl, n-propyl or tert-butyl.
"Alkenyl" means a straight-chain or branched-chain carbon group having one or more double bonds, preferably one double bond, preferably containing 2 to 16 carbon atoms, more preferably 2 to 8 carbon atoms, still more preferably 2 to 4 carbon atoms, e.g. ethenyl, propenyl or 1,4-butadienyl.
"Alkynyl" means a straight-chain or branched-chain carbon group having one or more triple bonds, preferably one triple bond, preferably containing 2 to 16 carbon atoms, more preferably 2 to 8 carbon atoms, still more preferably 2 to 4 carbon atoms, e.g. ethynyl or butynyl.
"Alkoxy" means a group of the structure R-O- wherein O is oxygen and R is an alkyl group as defined above.
"Cycloalkyl" means a cyclic alkyl group preferably containing 3 to 12 carbon atoms, more preferably 3 to 8 carbon atoms, still more preferably 3 to 6 carbon atoms, e.g. cyclopropyl or cyclohexyl.
"Aryl" preferably means a phenyl or naphthyl group.
"Heteroaryl" means a group having one or more aromatic rings containing one or more nitrogen, oxygen or sulphur atoms, e.g. thienyl or indolyl.
"Heterocyclyl" means a non-aromatic group having one or more rings containing one or more nitrogen, oxygen or sulphur atoms, e.g. piperazinyl, or tetrahydrofuryl.
"Halo" means a fluoride, chloride, bromide or iodide group.
"Carbonyl" means the -C(O)- group.
"Sulphonyl" means the -S(O)₂- group.
"Alkylthio" means a group of the structure R-S- wherein S is a sulphur atom and R is an alkyl group as defined above.

The present invention therefore involves the use of oligonucleotide aptamers as binding ligands to the tumour marker protein. The application of combinatorial chemistry coupled with polymerase chain reaction ("PCR") amplification techniques allow for the selection of small oligonucleotides from degenerate oligonucleotides libraries, which bind to target receptor molecules.

The use of aptamers as targeting agents offers several advantages compared with prior antibody techniques. The molecules are expected to penetrate the tumour much faster than whole antibodies, reach peak levels in the tumour sooner, and clear from the body faster thereby reducing toxicity to healthy tissues. In addition, the use of aptamers is expected to overcome the frequently encountered human anti-mouse antibody response. Also, the relatively small size of aptamers in comparison to whole antibodies favours their potential for greater tumour penetration, reduced immunogenicity, rapid uptake and faster clearance and makes them suitable vehicles for radionuclides and cytotoxic agents to be delivered to tumour. Aptamers that have very high affinity for targets are especially effective for a number of reasons. For example, a relatively small dose may be used in comparison with other treatments because the aptamers become localised in a target area, rather than being distributed throughout a body. Also, the effect of a therapeutic or diagnostic agent is enhanced by the high concentrations achieved within the target area.

A novel feature of the present invention is the use of a target which is bound to a support in the identification of an aptamer. More specifically, target MUC1 peptides were immobilised onto functionalised sepharose beads in a chromatography column. Binding aptamers are thus retained in the column with non-binding or weakly binding aptamers being washed off. The strongly binding aptamers may then be removed for amplification by PCR. The column selection/amplification steps can be repeated to distinguish the most strongly binding aptamer(s). It is to be appreciated that a different population of aptamers will be present at each successive cycle, and that a large population is present initially. The entire process can be repeated, for example, for ten successive rounds of selection and amplification, to effect affinity maturation through competitive binding. The resulting final aptamer(s) can be cloned and sequenced and successful aptamer(s) of high affinity and specificity identified. Other numbers of selection/amplification cycles could be used. This aspect of the invention is applicable to the identification of aptamers which bind to targets other than MUC1.

The strongly-binding aptamers of the invention may be used in a large number of ways. For example, they may be used in the treatment and/or prevention of diseases or conditions where expression of MUC1 occurs. They may also be used in the diagnosis or detection of such diseases and conditions, for example by in vitro or in vivo methods or tests. In particular, the aptamers of the invention may be used to direct other agents to the proximity of the target. Thus, an aptamer may be bound to an agent which kills or damages cells and/or which is detectable to locate concentrations of the target either in vitro or in vivo.

Elements such as yttrium, technetium and rhenium are useful in the detection and treatment of cancers and tumours; however the free ion is highly toxic. The use of a chelating ligand to form a stable complex has been used to overcome this problem and complexes of medicinally useful metals have been widely used in the diagnosis and treatment of cancer. They have the advantage that their diagnostic or therapeutic behaviour is often predictable as it is linked to some intrinsic property of the metal, for example radioactivity. Indeed, radioisotopes were among the first metals to be employed in clinical applications. While early examples of complexes of radioisotopes lacked tissue specificity, this problem is also overcome using a chelating ligand.

Ligands based on cyclen or porphyrins are an ideal scaffold for constructing chelating ligands for technetium and rhenium. Both cyclens and porphyrins have an arrangement of four nitrogens and derivatives have been shown to form stable complexes with metal ions in physiological conditions. It has the added advantage that it can be further functionalised with pendant arms to form a podand. Podands are a wide class of ligands with useful metal ion sequestering and binding properties. Novel cyclen derivatives are disclosed herein having a pendant methionine arm. This provides an additional sulphur donor atom to enhance the stability of the complex formed with rhenium and technetium. This creates a "lariat" type of ligand. The lariat ligand 9 disclosed herein uses the amines of the cyclen ring to coordinate the rhenium (IV) atom and the methionine arm wraps up the metal ion by coordination through the thioether group.

The carboxylate group of the methionine arm or on the porphyrin may be used as the point of attachment to a targeting aptamer. This group allows the use of a peptide coupling methodology to attach the complex via an amino group on the aptamer. In this way novel aptamers carrying radiolabels for tumour therapy may be produced. Such coupling methodologies are attractive as they proceed under mild conditions and allow multiple complexes to be loaded onto a single aptamer. In this way, higher local concentrations of the radioisotope can be achieved at the site of the tumour with a lower dose to reduce the side affects typically associated with radiotherapies.

A possible hindrance in the use of aptamers in therapy or as a diagnostic tool is their high cost and low bioavailability. In order to improve bioavailability and reduce cost, one needs to improve the bioavailability of the aptamer in circulation. The bioavailability (availability/stability in circulation) is dependent on two main factors; degradation time (half life) and size. The half life of an aptamer can be easily tailored by the addition of specifically modified bases that protect it against degradation from naturally occurring nucleases, which can also be chosen to further functionalize the molecule. Size is another important factor in the availability of aptamers. Aptamers may be designed to be small molecules to allow for better tumour penetration and specificity, but when in circulation, their size allows the molecules to be readily filtered out through the normal mechanisms of the body, by rapid tissue diffusion and renal clearance. The present invention seeks to address this problem by functionalisation of the basic aptamer molecule. In particular, a strategy is proposed which may achieve the required size on size factor using a novel method to overcome this problem that also allows further functionalization of the aptamer construct and minimizes immune reactions problems, whilst potentially increasing the aptamer's imaging capability.

Affinity based screening was used to screen a short DNA library of aptamers which bind to MUCI targets with an exquisite specificity and high avidity. These aptamers were further modified to resist endonucleases when given as a therapy. The resulting family of aptamers has an average size of 10,000 Kda, thus facing the challenge of staying in circulation long enough to allow for binding of the molecules to the MUCI target and so to become an effective therapy due to their small size.

The strategy disclosed herein comprises the functionalization of aptamers via the attachment to a macrocyclic molecule; DOTA is a suitable macrocycle that has four equally spaced reactive carboxyl arms. These arms are able to react swiftly and effectively with the amino functionality of aptamers or modified aptamers with the addition of a coupling agent; such as EDCI (1-(3-dimethylaminopropyl)-3-ethylcarbodiimide). Aptamers may be designed to contain amino modifications at only the 3' or at both the 3' and 5' ends. The reactive carboxylic arms of DOTA can react with the reactive amino modifications in a simple step. Furthermore, the end of the aptamer that is left unreacted can be further functionalised with the bifunctional chelator S-Acetyl-NHS-MAG3 to enable the resulting aptamer construct to carry five metal atoms because it carries five chelator sites (4 MAG molecules and the reactive center of DOTA). This complex should have greater resistance to degradation and better bioavailability, as well as carrying a greater amount of radioisotope per molecular construct, and thus be more efficient as a therapeutic and/or diagnostic tool.

The strategy of forming multivalent aptamers allows for the creation of heterogenous as well as homogeneous complexes. For example, the central chelating site of the DOTA residue may be occupied by gadolinium whilst the terminal coordination sites may be occupied by radioactive isotopes. This combines both imaging (through MRI using the gadolinium) and cytotoxicity through the radioisotopes. This strategy allows the in vivo monitoring of the biodistribution of any radio or chemotherapy using conventional MRI scanners. In other words, such complexes allow for a 'dual mode' of operation. The distribution of the complexes can be monitored by using one attached agent, allowing detection or diagnosis, whilst the complexes may also radiate tumours etc with another attached agent. A 'dual mode' of both monitoring and therapy is thus possible.

The labelling of the complexes can be verified using a range of physical techniques such as absorption spectroscopy, mass spectrometry, and in the case of fluorescent labels such as rhodamine and fluorescein, by fluorescence spectroscopy, and by relaxometry for MRI active labels.

The invention will be described further with reference to generalised synthetic schemes and protocols.

### A: Synthesis of Ligands

As mentioned above, novel ligands were synthesised. Scheme 1 below shows the synthesis of a methionine moiety which was to be coupled to a ring:

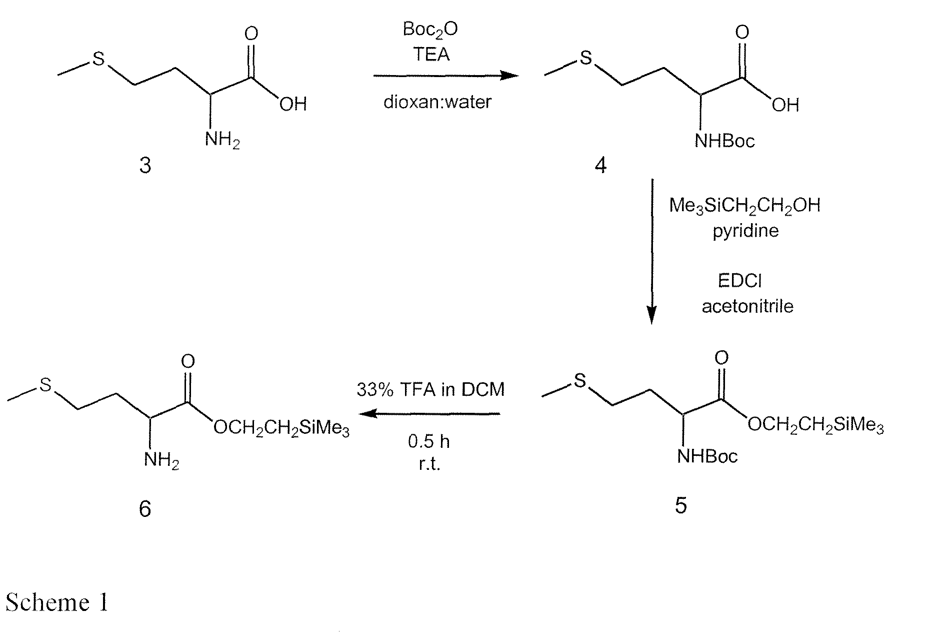

The amino group of commercially available D,L-methionine 3 was protected with a tert-butoxycarbonyl (Boc) group to give 4 in very good yield.

The carboxylic acid group of 4 was then protected as a 2-trimethylsilylethyl ester 5 in good yield, followed by removal of the Boc group to give the methionine moiety 6.

The compound 6 was then coupled with a cyclen compound, as shown in scheme 2 below.

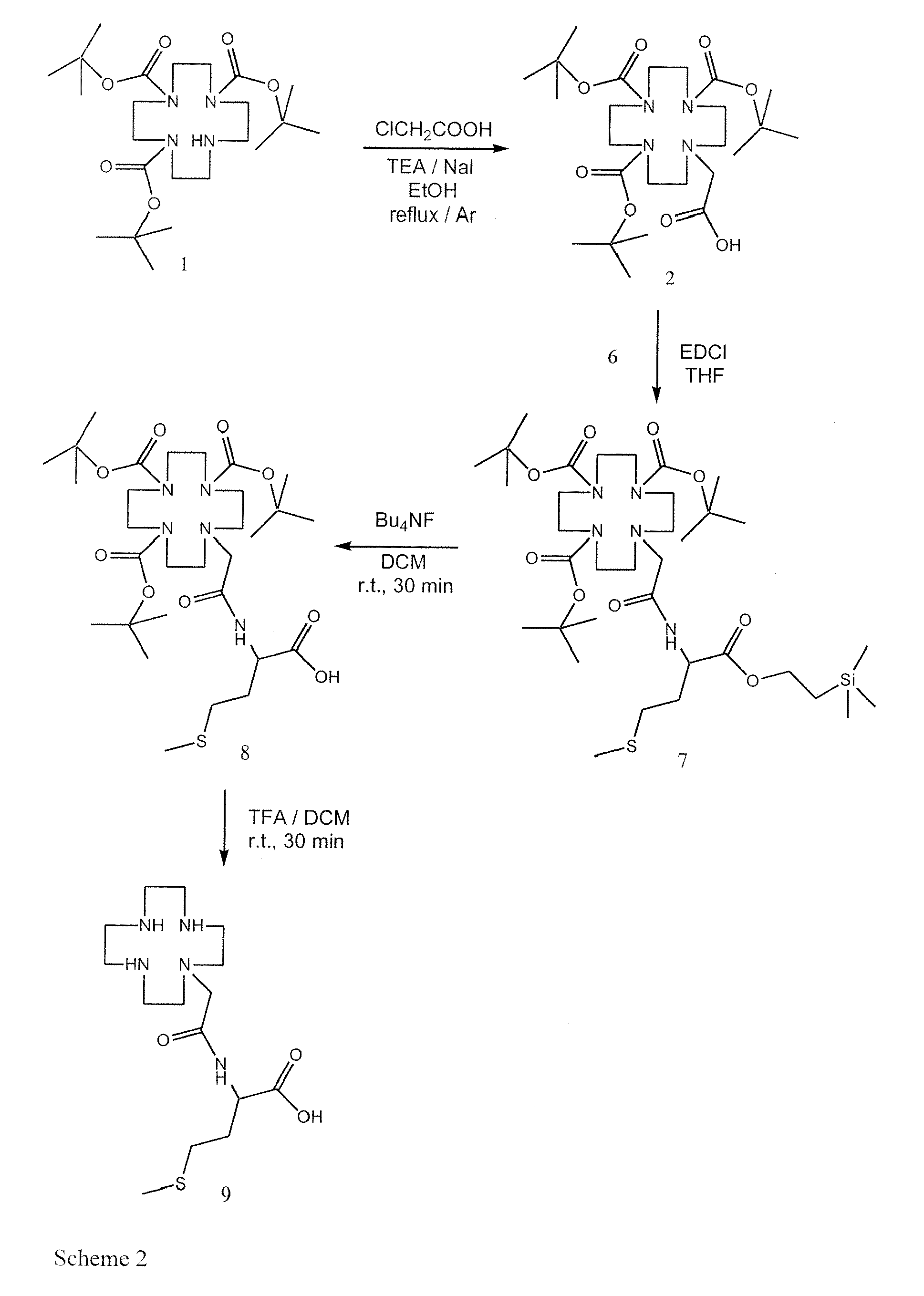

Tri-Boc cyclen 1 was reacted with chloroacetic acid to give 2, which was coupled with the methionine derivative 6 to give 7 in quantitative yield. Removal of the silyl protecting group gave 8, with subsequent removal of the Boc groups giving ligand 9.

In addition to the published synthesis of tri-Boc cyclen 1, it could also be prepared by treatment of cyclen 17 with Boc anhydride and triethylamine, as shown below in scheme 3.

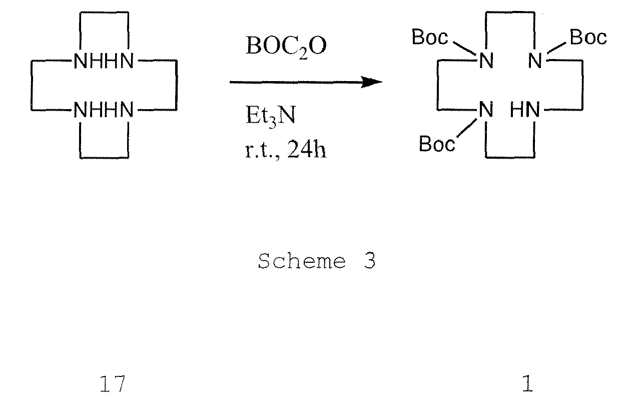

An alternative and more efficient synthesis of ligand 9 is shown below in scheme 4.

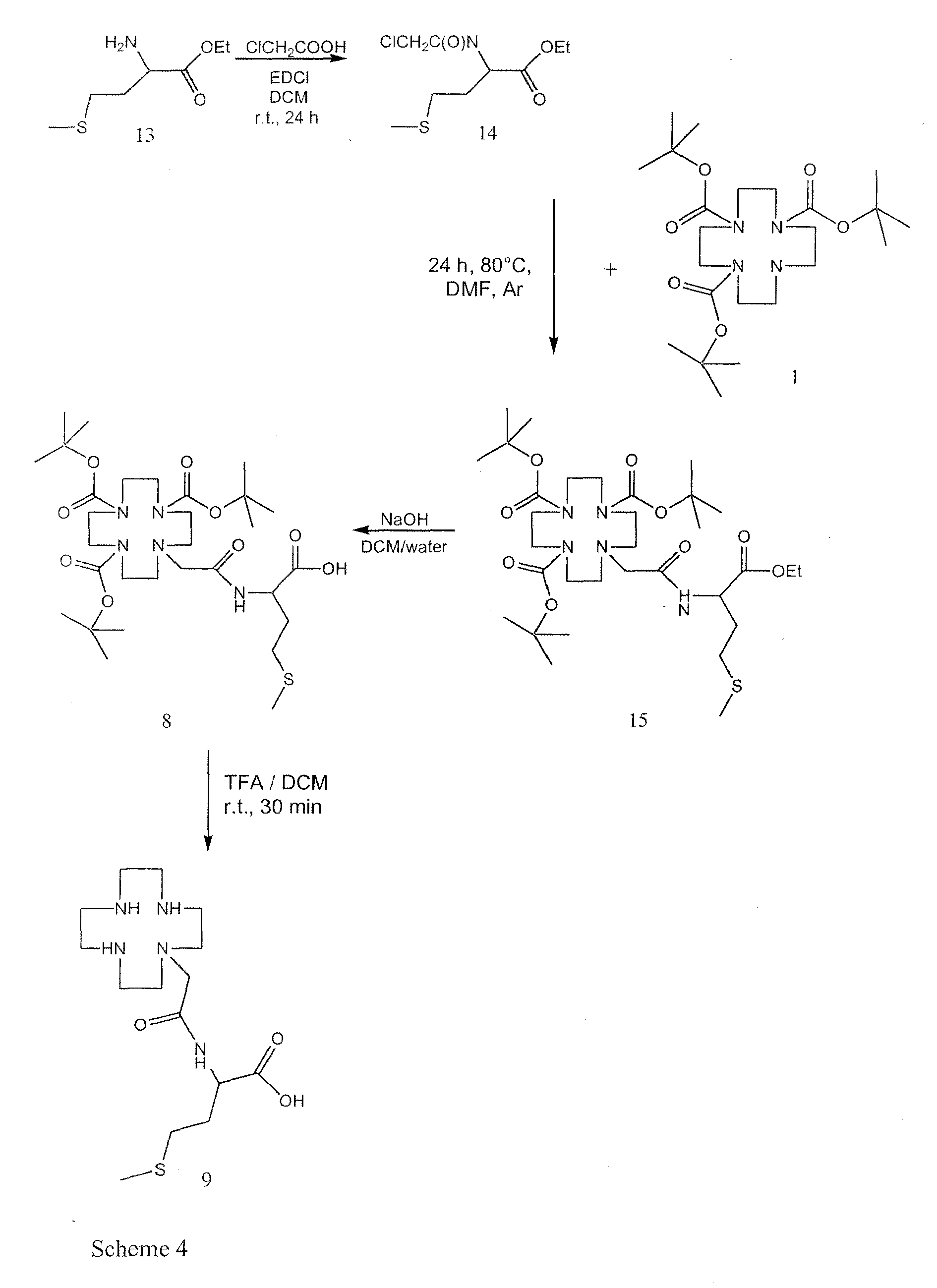

Commercially available D,L-methionine ethyl ester 13 was reacted with chloroacetic acid to give the amide 14 in very good yield. The amide 14 was coupled with tri-Boc cyclen 1 to give the compound 15. Ester hydrolysis gave the acid 8 which was then deprotected by removal of the Boc groups to give ligand 9.

Forphyrin-based ligands were also synthesised as set out below in scheme 5.

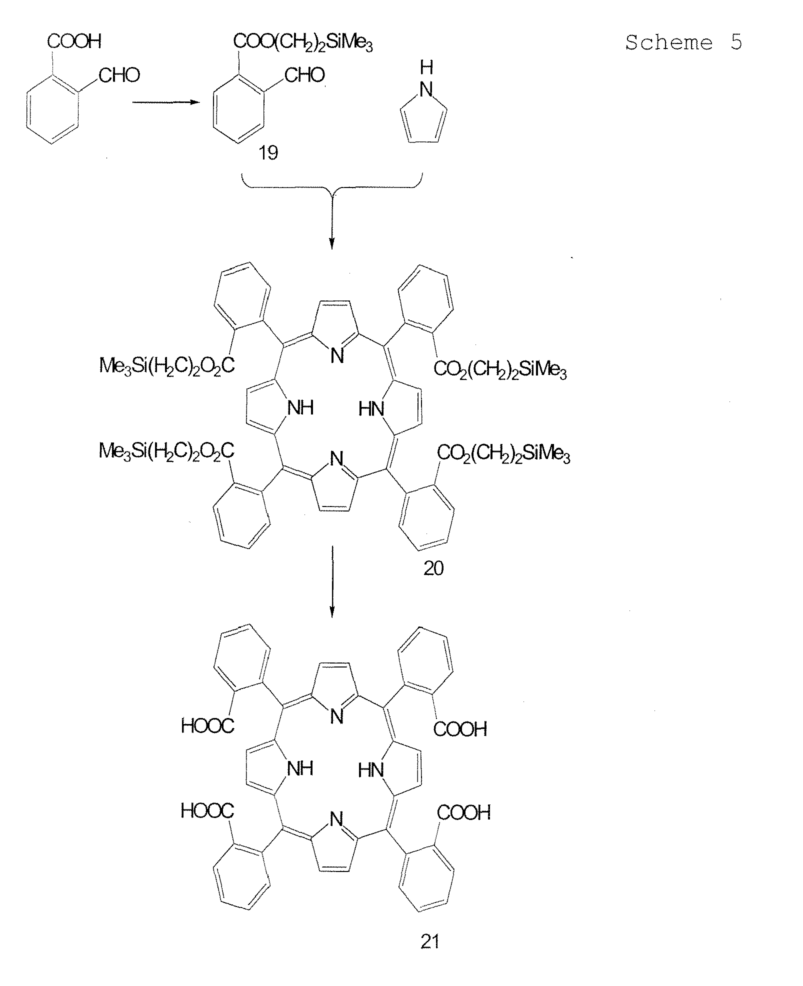

Commercially available 2-carboxy benzaldehyde was converted to the ester 19 in good yield. Ester 19 was reacted with pyrrole to give the porphyrin compound 20. Deprotection of compound 20 gave the tetra-benzoic acid porphyrin compound 21 in quantitative yield.

Ligands such as compound 9 may then be complexed with a metal as shown below in scheme 6.

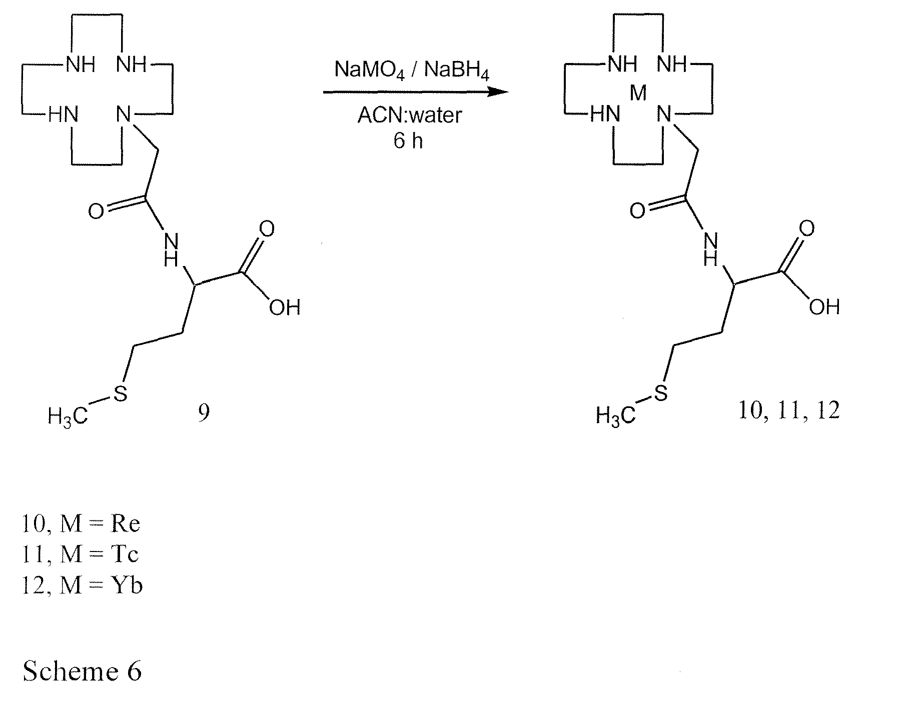

Ligand 9 was treated with sodium perrhenate under reducing conditions to give the rhenium-containing complex 10. Similar reactions give the technitium complex 11 and the yttrium complex 12.

Thus, a functionalised aptamer may have one attached ligand, as schematically shown below:

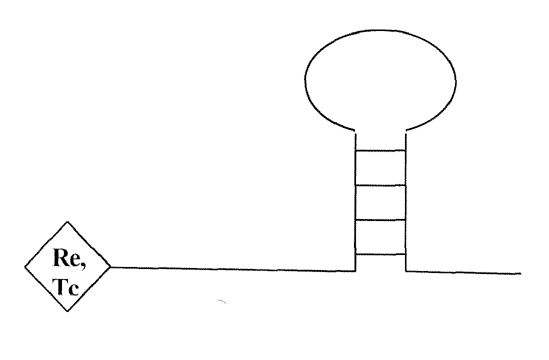

As discussed above, however, it is possible to attach multiple ligands to an aptamer and/or attach multiple aptamers to a ligand. A unit comprising five ligands and four aptamers is schematically shown below: 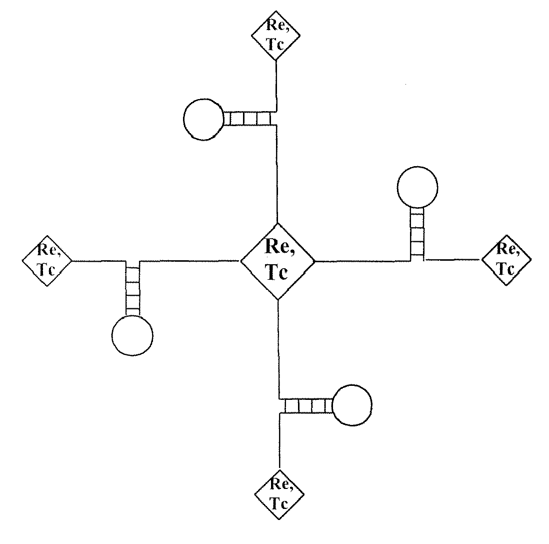 Amino modified aptamers with modification at both the 3' and the 5' end are used. Four aptamer recognition units are involved, which are attached via peptide bonds to the four carboxy groups of DOTA using a standard peptide coupling reaction with starting materials of excess aptamers (≥4:1 of aptamer to DOTA) to allow for coupling to all available coupling sites. MAG3 (or any other ligand, such as ligand 9 or other commercial ligands) is then coupled to the other end of the aptamer, resulting in a four-aptamer complex carrying effectively 5 ligands loaded with radionuclides.

This novel method of labelling increases the amount of radiation that may be taken to the cell target, reduces the susceptibility of the aptamer to nucleases (enzymes that chop up nucleotides including aptamers) and increases the half-life of the aptamer, allowing it to remain active in the body. By protecting both ends of the aptamer through linking them to the radionuclide carrying ligands, additional nuclease resistance can be obtained. Furthermore, due to their small size, unmodified aptamers are cleared very quickly from the system through the kidneys, thus leaving little useful time in circulation. By linking four aptamers together, the molecule is effectively increased in size (about 40kDa in total, instead of 10kDa for each individual unit), thus limiting its clearance from the system and offering additional useful time in circulation. The circulation time of such modified aptamers may be several hours, matching or surpassing the half-life of the relevant radionuclide.

Modified aptamers coupled to the MAG3 ligand are shown below in figure 10, (with the aptamers themselves not being shown but which are linked to the sugar residue).

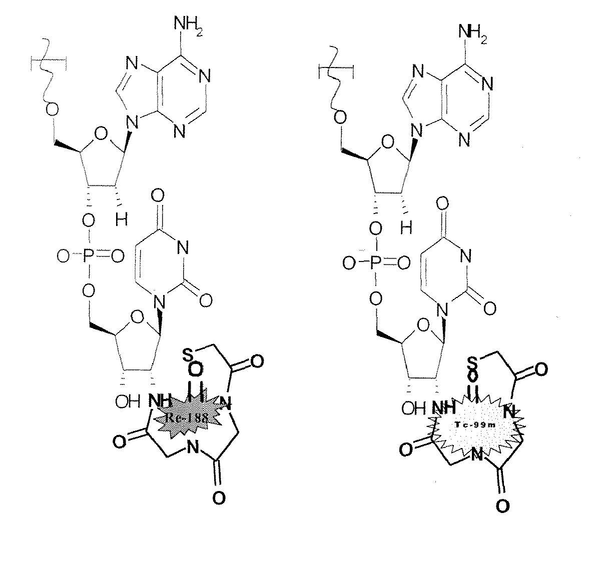

### B: Generation, selection and identification of aptamers

MUC1 peptides were purchased from the Biopolymer Synthesis and Analysis Unit, Nottingham University, Nottingham, UK. Two peptide sequences were synthesised, one was a triple repeat of the human MUC1 tandem repeat peptide APDTRPAPGSTAPPAHGVTS and the other was the nine amino acid peptide epitope that forms part of this sequence, namely the APDTRPAPG.

Single stranded DNA libraries consisting of a 25-base variable region comprising of equimolar nucleotides addition flanked by a 23-base and a 24-base primer were chemically synthesised and HPLC purified, (Biopolymer Synthesis and Analysis Unit, Nottingham University, Nottingham, UK). A list of computer based programs for the correct design of primers for use in this reaction can be found at:
http://seqcore.brcf.med.umich.edu/doc/dnaseq/primers.html
http://www.hgmp.mrc.ac.uk/GenomeWeb/nuc-primer.html
http://bioinformatics.weizmann.ac.il/mb/bioguide/pcr/software.html

A variable region of 25 bases was chosen as this is thought to be the smallest length which can give all possible three dimensional structures such as hyper-loops and G-quadruplexes etc. Also, a 25 base variable region gives a theoretical sequence space of 4²⁵ (i.e. around 1 x 10¹⁵), which is a practical population of aptamers to actually prepare in a routine experiment. Thus, whilst the present application discloses variable oligonucleotide sequences of 25 bases or less, it is to be appreciated that longer sequences which bind to targets (e.g. MUC1 polypeptides) may contain some or all of the oligonucleotide sequences disclosed herein.
Library: 5'>Primer 1 (23 bases) - Variable region (25 bases) - Primer 2 (24 bases)<3'
Library: 5'> GGGAGACAAGAATAAACGCTCAA- N25-TTCGACAGGAGGCTCACAACAGGC<3' (concentration: 40µM)
Forward Primer 5'>GGGAGACAAGAATAAACGCTCAA (concentration: 40µM)
Reverse Primer 5'>GCCTGTTGTGAGCCTCCTGTCGAA<3' (concentration: 1mM)

*In vitro selection*: Standard procedures for in vitro selection or SELEX experiments, described at Science 249 (4968) 505-510 (1990), and Nature (London), 346 (6287) 818-822 (1990) were followed throughout, with a number of modifications and improvements as described below. Fragments of target MUC1 sequence with known immunogenic responses as described above were bound to a Hi trap column (NHS activated) (selection column, provided by Pharmacia Biotech) according to manufacturer instructions. The column forms a covalent bond with compounds having a primary amino group, such as a terminal amino group of a polypeptide. The pools of DNA templates (the library) were added to the chromatography column and let interact with the target peptide for approximately 1-hour at room temperature. The column was washed to remove any unbound aptamers and the bound aptamers were eluted with elution buffer (3M sodium thiocyanite). The eluted samples were then desalted with a NAP-10 column (provided by Pharmacia Biotech) and finally eluted in sterile water in an eppendorf. These were subsequently freeze-dried and polymerase chain reaction ("PCR") reagents were added to the dry oligonucleotides to prepare them for the PCR, which was performed for 99 cycles with an annealing temperature of 56°C. After the PCR procedure the DNA generated from this amplification was added to the chromatography column and used for the next selection round. These successive rounds of selection and amplification were carried out for 10 times. The final product achieved was a PCR product of about 100µl.

*Characterisation of clones:* After 10 rounds of selection and amplification, the pool was cloned to screen for DNA molecules with affinity for MUC1 (TA Topo cloning Kit, Invitrogen, UK). Individual clones were characterised using a general PCR protocol, with annealing temperature of 48°C, for 35 cycles using M13 primers, and visualised on a 2.5% Agarose gel. The positive clones were later grown in LB media in the presence of Ampicillin and isolated using a standard plasmid DNA isolation kit (Quiagen, UK).

The pool was further sequenced using standard IRD-800 radioactive method (Sequitherm EXCEL II, Epicentre Technologies, Madison, USA).

### Results

*Isolation of DNA aptamers:* In vitro selection began with a pool of DNAs with a random sequence of 25 nucleotides flanked by two primer-binding regions. The DNAs in the binding buffer were loaded onto the Hitrap NHS-activated column. The unbound or weakly bound DNAs were washed from the column. The DNAs that specifically bound to the MUC1 moiety were affinity-eluted with elution buffer and further desalted with a NAP-10 column, freeze dried and amplified by PCR. The fraction eluted from the 10th round of amplified DNA were collected and double stranded for subsequent cloning. The pooled DNA was analysed on a 2.5% Agarose gel. The dsDNAs were cloned using TA Topo cloning kit and the DNA plasmid was analysed and quantified on a 1% Agarose gel. Several individual clones were sequenced from both the selections, the one using the nine amino acid residues and the other using the sixty amino acid residues MUC1 peptides.

The sequences identified from the selection of aptamers using the nine-residue peptide APDTRPAPG were as follows:
1 CGAATGGGCCCGTCCTCGCTGTAAG
2 GCAACAGGGTATCCAAAGGATCAAA
3 GTTCGACAGGAGGCTCACAACAGGC
4 TGTTGGTCAGGCGGCGGCTCTACAT
5 XXCTCTGTTCTTATTTGCGAGTTXX

A CCCTCTGTTCTTATTTGCGAGTTCA
B CTCTGTTCTTATTTGCGAGTTGGTG
C CCCTCTGTTCTTATTTGCGAGTTCA
D TAAGAACAGGGCGTCGTGTTACGAG
E GTGGCTTACTGCGAGGACGGGCCCA
F GCAGTTGATCCTTTGGATACCCTGG
G AACCCTATCCACTTTTCGGCTCGGG

When the clones were analysed, the above sequences were identified as binding to both the MUC1 9mer and the MUC1 60mer peptides. Both the MUC1 9mer and MUC1 60mer peptides resulted in the same binding sequences. The first four sequences seem to have a total of 25 bases epitope which was conserved in all the clones analysed. However, sequence number 5 appeared to require only a 21 base epitope which was shifted upstream and downstream within the variable region of the aptamer, tolerating all other combinations at each end.

In the above sequence 5, X designates any base, such as C, G, A or T. Sequences A, B and C show specific examples of such variations within sequence 5.

These sequences 3 and 4 appear in the sequencing experiments associated with the primers flanking the variable region in the library. The sequences 3 and 4 were sequenced in the forward direction and were found in the form of:

5'>GGGAGACAAGAATAAACGCTCAAGTTCGACAGGAGGCTCACAACAGGC
TTCGACAGGAGGCTCACAACAGGC<'3 and

5'>GGGAGACAAGAATAAACGCTCAATGTTGGTCAGGCGGCGGCTCTACAT
TTCGACAGGAGGCTCACAACAGGC<'3

On the other hand, the sequences 3 and 4 were identified from a complementary single stranded DNA containing the reverse primer and the sequence identified for the variable region.

Thus, the actual oligonucleotides which were isolated using the method of the invention included the primers and so had the full structures given below:

1      5'>GGGAGACAAGAATAAACGCTCAACGAATGGGCCCGTCCTCGCTGTAAG
TTCGACAGGAGGCTCACAACAGGC<'3

2      5'>GGGAGACAAGAATAAACGCTCAAGCAACAGGGTATCCAAAGGATCAAA
TTCGACAGGAGGCTCACAACAGGC<'3

3      5'>GGGAGACAAGAATAAACGCTCAAGTTCGACAGGAGGCTCACAACAGGC
TTCGACAGGAGGCTCACAACAGGC<'3

4 5'>GGGAGACAAGAATAAACGCTCAATGTTGGTCAGGCGGCGGCTCTACAT

TTCGACAGGAGGCTCACAACAGGC<'3

5     5'>GGGAGACAAGAATAAACGCTCAAXXCTCTGTTCTTATTTGCGAGTTXX
TTCGACAGGAGGCTCACAACAGGC<'3

A     5'>GGGAGACAAGAATAAACGCTCAACCCTCTGTTCTTATTTGCGAGTTCA
TTCGACAGGAGGCTCACAACAGGC<'3

B 5'>GGGAGACAAGAATAAACGCTCAACTCTGTTCTTATTTGCGAGTTGGTG
TTCGACAGGAGGCTCACAACAGGC<'3

C     5'>GGGAGACAAGAATAAACGCTCAACCCTCTGTTCTTATTTGCGAGTTCA
TTCGACAGGAGGCTCACAACAGGC<'3

D     5'>GGGAGACAAGAATAAACGCTCAATAAGAACAGGGCGTCGTGTTACGAG
TTCGACAGGAGGCTCACAACAGGC<'3

E     5'>GGGAGACAAGAATAAACGCTCAAGTGGCTTACTGCGAGGACGGGCCCA
TTCGACAGGAGGCTCACAACAGGC<'3

F     5'>GGGAGACAAGAATAAAACGCTCAAGCAGTTGATCCTTTGGATACCCTGG
TTCGACAGGAGGCTCACAACAGGC<'3

G 5'>GGGAGACAAGAATAAACGCTCAAAACCCTATCCACTTTTCGGCTCGGG
TTCGACAGGAGGCTCACAACAGGC<'3

The relatively high salt condition of the above-mentioned examples were used to ensure only high affinity aptamers were selected for the targe (MUCI). However, given the fact that *in vivo* conditions do not include such high salt buffer conditions, further aptamer selection experiments were performed procedures previously described, but using a start buffer (binding buffer) of 0.1M NaCl and 0.005M MgCl₂ which more closely simulates physiological conditions. This selection resulted in the sequences H and I listed below.
H AGAAACACTTGGTATATCGCAGATA
I GGGAGACAAGAATAAACACTCAACG

Also, further selection experiments were performed using the mutated MUCI APDTREAPG peptide. Further selection experiments were performed using the exact protocols and procedures describes above for aptamers 1 to 5 and A to G, to give aptamers J, K and L listed below:
J CGATTTAGTCTCTGTCTCTAGGGGT
K CGACAGGAGGCTCACAACAGGCAAC
L AGAACGAAGCGTTCGACAGGAGGCT

With this information, compounds may be directly synthesised which comprise these entire sequences or a part thereof, in particular the variable region. Also compounds may be made with sequences having homology to these sequences which have a good binding affinity. Also, compounds may be made which have a modified sequence to those shown. Such modifications may include the use of unnatural bases or the use of modified natural bases. Also, it is to be appreciated that longer sequences may be prepared that comprise one or more of the sequences listed above, or a variation or modification thereof, whilst still retaining the ability to bind to MUC1.

Oligonucleotide synthesis may be performed using a solid-phase phosphoramidite synthesis with an automated synthesiser (such as those made by Abi Applied Biosystems, Foster City, California, USA). Experimental information may be found in the following references: Beaucage, S.L. and Caruthers, M.H., Tetrahedron Letters (1981), 22, 1859 and Matteucci, M.D., and Caruthers, M.H., J. Am. Chem, Soc. (1981), 103, 3185.

The aptamers may comprise modifications including amino, fluoro, methyl, phosphate and/or thiol modifications at the 3' and 5' ends. The modifications may be to the phosphate, sugar and/or base of each nucleotide. Examples of modifications include 6-substituted purine nucleotides (US 3915958), fluorescent derivatives of adenine-containing compounds (US 03960840), 8-substituted cyclic nucleotides (US 3968101), 6-aminocarbonyl purine 3', 5'-cyclsc nucleotides (US 4038480), 8-substituted cyclic adenosine monophosphate derivatives (US 4048307), C-5 substituted cytosinenucleosides (US 4267171) and cytidine nucleotide derivatives (US 4086417). Many modified and unnatural nucleotides are commercially available from companies such as Jena Bioscience GmbH, Jena, Germany, including fluorescent *mant*-nucleotides (*mant* = 2'/3'-O-(N-methylanthraniloyl), *caged*-nucleotides (carrying a photo-labile group), non-hydrolysable nucleotides, xanthosine and inosine nucleotides and halogen-containing nucleotides.

An example of such a modification is shown below in scheme 7. A sugar group of a nucleotide at the 3' end of a sequence is modified by the replacement of a hydrogen with an amino group. The amino group is particularly useful as it allows the sequence to be connected to another moiety by coupling with a carboxyl group.

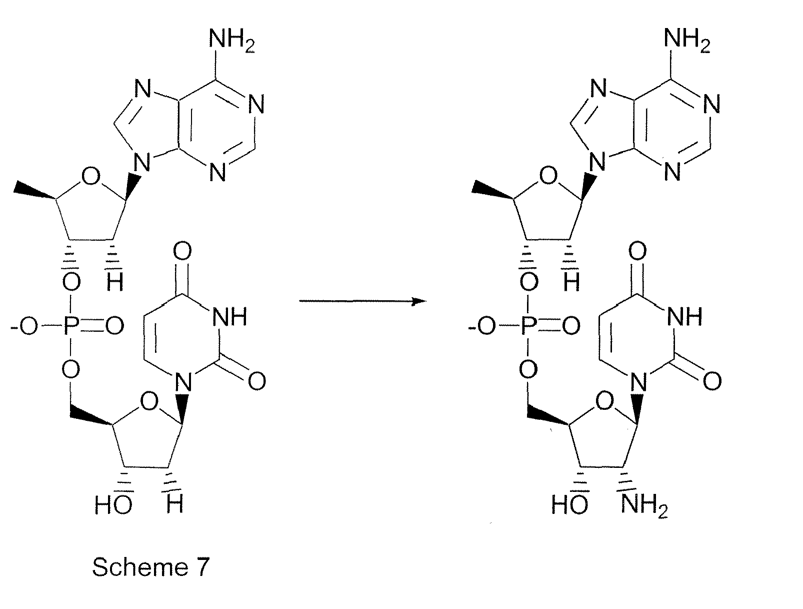

Further examples of possible modifications are shown below in scheme 8, along with possible uses and advantages.

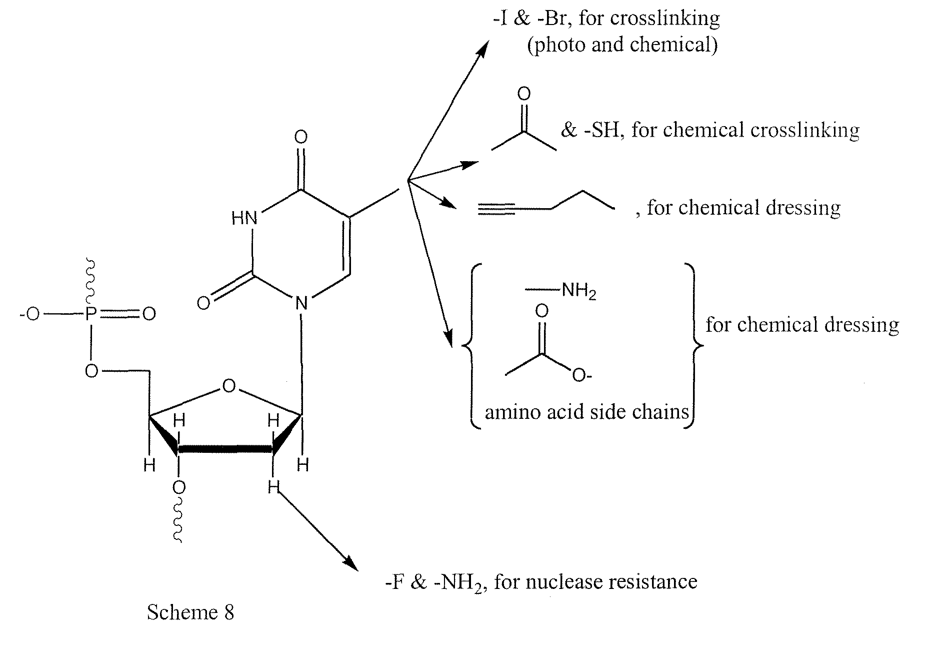

The aptamers of the invention, or variations thereon, may be connected to another compound for various uses, such as therapy or diagnosis. An aptamer may be joined to a ligand, such as those disclosed herein, by, for example, ionic or covalent bonds, or by other ways such as hydrogen bonding. The aptamer may thus guide the ligand to the target. The aptamer is preferably directly connected to the ligand. More specifically, the aptamer may be bound to the ligand without the use of a peptide tether. An aptamer may be joined to a ligand or other agent by a pendant moiety such as an amino or hydroxyl group. Several other agents may be attached to the same aptamer, and several aptamers may be attached to the same agent.

The aptamers could be linked to ligands such as MAG2 (mercaptoacetyl diglycerine), MAG3 (mercaptoacetyl triglycerine), HYNIC (hydrazinonicotinic acid), N₄-chelators, hydrazino-type chelators and thiol-containing chelators. In particular, DOTA and related cyclen derived ligands are suitable for functionalising aptamers. Also, the aptamer could be linked to fluorescent or phosphorescent groups and MRI agents. Examples include fluorescein, rhodamine, biotin, cyanine, acridine, digoxigenin-11-dUTP, and lanthanides.

The aptamer labelling may be carried out using standard peptide coupling protocols. A sample labelling experiment is given below:

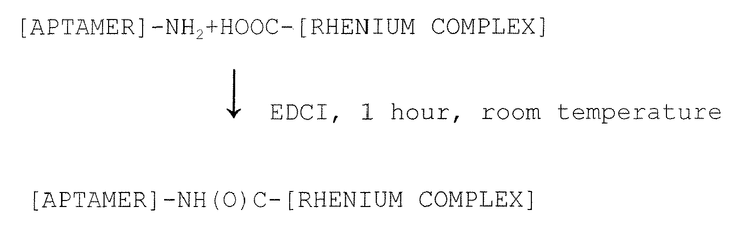

0.01 mmol (0.004 g) of compound 11 or 0.01 mmol (0.009 g) porphyrin was dissolved in 0.5 cm³ water and 0.5 cm³ DMF. 0.002 g EDCI was added to the solution, which was stirred at room temperature for 15 min. 1 equivalent of the aptamer in 1 cm³ water was added and the reaction was allowed to proceed for 1 hour. The sample was applied onto a gel filtration column (Nap-10) and the conjugate was eluted with 12 cm³ PBS (phosphate buffer saline). 1 cm³ fractions were collected, and the fractions containing the conjugate were combined.

The porphyrin ligands used in the labelling protocol described above are obtained commercially or synthesised using established methods such as those described in Tetrahedron, 1997, 53, 6755-6790.

Radiolabelled aptamers may be prepared for targeting purposes. In order to evaluate the efficacy of aptamers as therapeutic or diagnostic agents, the ligand would be loaded with the radionuclide as it comes off the generator and then coupled to the aptamer and administered immediately. Alternatively, the ligand may be first coupled to the aptamer and then only loaded with the radionuclide prior to administration. Monitoring under a gamma-camera after each administration and during the course of a treatment will provide evidence of the efficacy of the aptamer as a diagnostic and therapeutic reagent.

It is to be appreciated the methodology of the invention is not limited to DNA aptamers. It is also applicable to other types of oligonucleotides, such as RNA and oligonucleotides comprising modified moieities, such as unnatural bases or modified natural bases.

The invention will now be described with reference to specific examples and detailed protocols:

### Experimental Details

### Materials and Reagents

All materials are either commercially available or have published syntheses. For example, tri-Boc cyclen may be synthesised as described in J. Am. Chem. Soc. 1997, 119, 3068-3076. The target MUC1 peptides were purchased from Biopolymer Synthesis and Analysis Unit, Nottingham University, Nottingham, UK. The DNA library of <primer>-<25 variable bases>-<primer> oligonucleotides was ordered from Biopolymer Synthesis and Analysis Unit, Nottingham University, Nottingham, UK. Standard methodologies were used in parts of the experiments, such as those described in "The Practice of Peptide Synthesis", M. Bodansky, A. Bodansky, 1984, Springer-Verlag, Berlin.

### A: Synthesis of Ligands

### Synthesis of tri-Boc cyclen

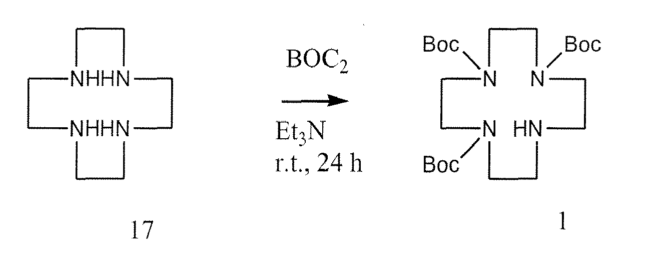

Cyclen (17) (412 g, 2.4 mmol) was dissolved in 25 mL chloroform, 1.53 mL triethylamine was added, and the solution was vigorously stirred at room temperature while Boc-carbonate (1.72 g, 7.89 mmol, dissolved in 25 mL chloroform) was introduced in 4 hours. The reaction was allowed to proceed for 24 hours, then the solvent was removed in vacuo. The residue was dissolved in 3 mL chloroform, and applied onto a silica column. Chromatography with ethyl acetate : dichloromethane (4:6) afforded the triBoc cyclen (18) as a white solid. Yield 0.86g, 76%.

### Alkylation of tri-Boc cyclen with chloroacetic acid

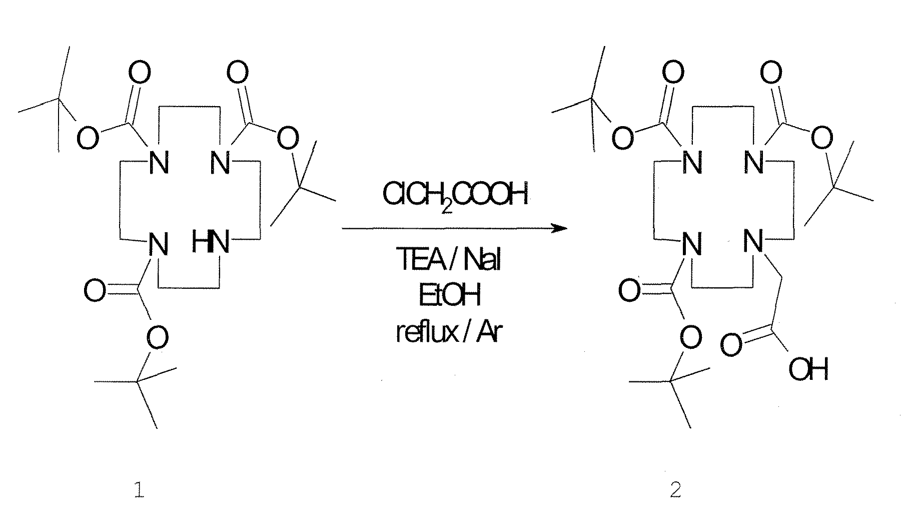

0.24 g (0.5 mmol) tri-Boc cyclen (1) was dissolved in 10 cm³ 100% ethanol, 0.5 g (5.3 mmol) chloroacetic acid, 1.5 g (15 mmol, 1.56 cm³) triethylamine and 0.5 g (3.3 mmol) sodium iodide were added and the mixture was refluxed under an argon atmosphere for 24 hours. The ethanol and the triethylamine were removed *in vacuo,* 30 cm³ dichloromethane and 40 cm³ water were added to the residue, the phases were separated and the water phase was extracted with 2 x 25 cm³ dichloromethane. The combined organic phases were washed with dilute acid (0.1 M hydrochloric acid, 25 cm³) and water (25 cm³), dried over MgSO₄, filtered and evaporated to dryness to give (2) as a yellow oil. Yield: 0.19 g, 70%.

### Protection of the amino group of Boc-D,L-Methionine

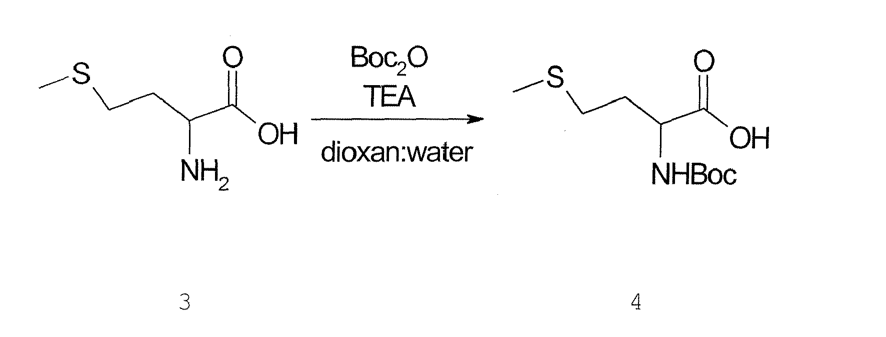

1.49 g (10 mmol) D,L-methionine was dissolved in 6 cm³ water and 6 cm³ 1,4-dioxan, 2.1 cm³ triethylamine (15 mmol) and 2.42 g (11 mmol) di-tert-butyl dicarbonate were added and the reaction mixture was stirred at room temperature for three hours. 25 cm³ ethyl acetate and 25 cm³ distilled water were added, the phases were separated and the water phase was acidified to pH 2 with 1:1 aqueous hydrochloric acid. The product was extracted into ethyl acetate (3 x 25 cm³), the organic phase was dried over MgSO₄, filtered and evaporated to dryness. The residue was dried *in vacuo* to give a white solid. Yield: 2.29 g, 92%.

### Synthesis of N-Boc-D,L-Methionine 2-trimethylsilylethyl ester

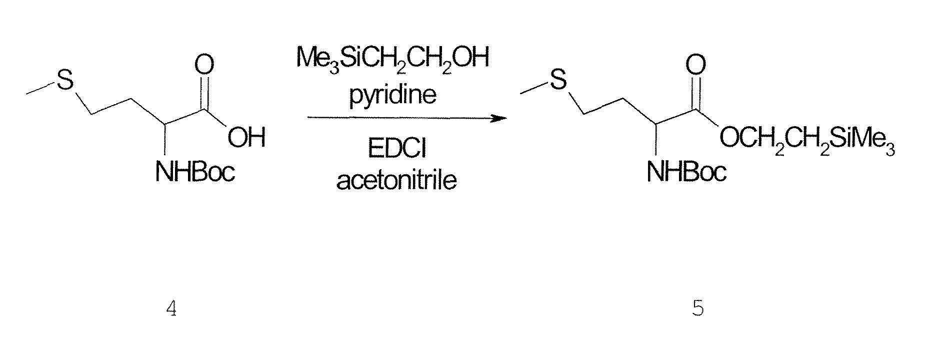

0.49 g (1.97 mmol) N-Boc-D,L-methionine 4 was dissolved in 8 cm³ acetonitrile, the solution was treated with 0.8 cm³ (1.97 mmol) pyridine and 0.59 g (1.97 mmol) 2-trimethylsilylethanol and stirred at room temperature for 10 minutes. 1.06 g (5.4 mmol) EDCI was added to the solution and the reaction was allowed to proceed for 12 hours. The acetonitrile was evaporated, the residue was dissolved in 25 cm³ ethyl acetate and 25 cm water, the phases were separated and the water phase was extracted with 2 x 20 cm³ ethyl acetate. The combined organic phases were washed with dilute base (0.5 M NaOH solution, 25 cm³) and water (25 cm³), dried over MgSO₄, filtered and evaporated to dryness to give a colourless oil. Yield: 0.50 g, 73%.

### Deprotection of D,L-Methionine 2-trimethylsilylethyl ester

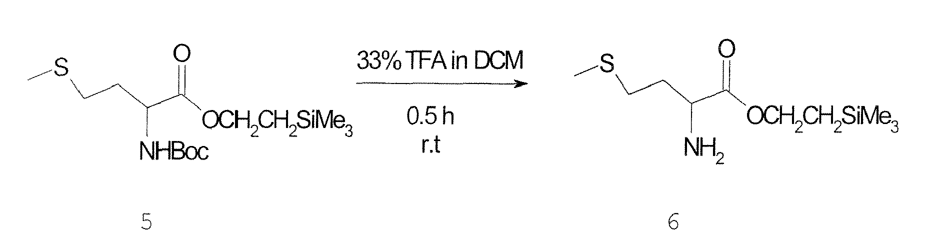

0.50 g (1.43 mmol) N-BoC-D,L-Methionine 2-trimethylsilylethyl ester 5 was dissolved in 10 cm³ dichloromethane, 5 cm³ trifluoroacetic acid was added and the solution was stirred at room temperature for half an hour. Sodium hydrogen carbonate was added until no more gas evolved, the suspension was dissolved in 30 cm³ water and 20 cm³ dichloromethane, the phases were separated, the pH of the water phase was adjusted to 12 and the product was extracted into DCM with 3 x 25 ml. The combined organic phases were washed with dilute base (0.5 M NaOH) and water (25 to 25 cm³), dried over MgSO₄, filtered and evaporated to dryness to give a colourless oil. Yield: 0.11 g, 30%.

### Coupling D,L-Methionine-2-trimethylsilylethyl ester to N¹, N⁴, N⁷-tri-Boc-N¹⁰-carboxymethyl cyclen

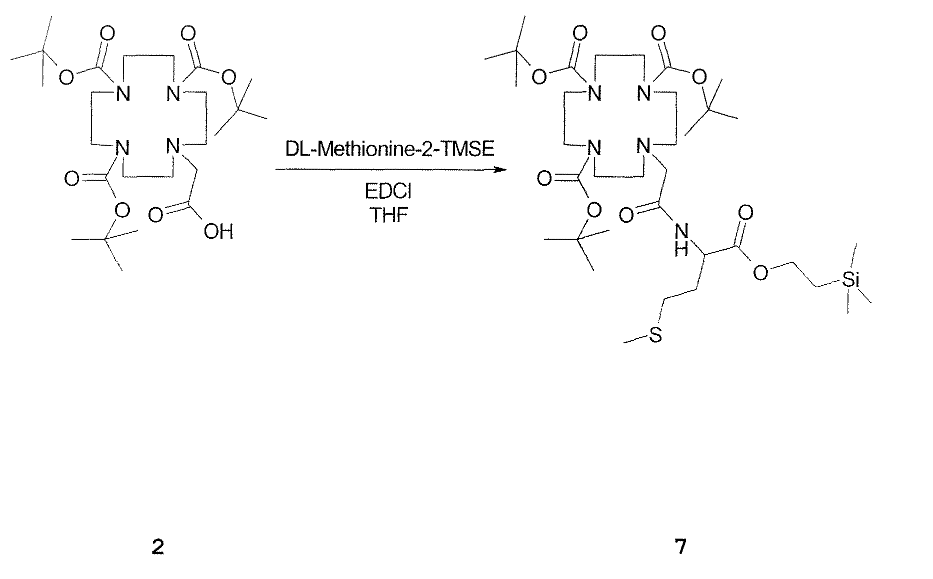

0.27 g (0.5 mmol) N¹, N⁴, N⁷-tri-Boc-N¹⁰-carboxymethyl cyclen 2 was dissolved in 15 ml tetrahydrofuran, 0.25 g (1 mmol)D,L-Methionine-2-trimethylsilylethyl ester 6 was added and 0.19 g (2 mmol) EDCI. The THF was evaporated, the residue dissolved in 40 cm³ dichloromethane, and the excess methionine, EDCI and the urea were washed away with 2 x 25 cm³ water. The organic phase was dried over MgSO₄ and the solvent was evaporated *in vacuo* to give a yellow oil. Yield: 0.35 g, 98%.

### Removal of the 2-trimethylsilylethyl ester protection

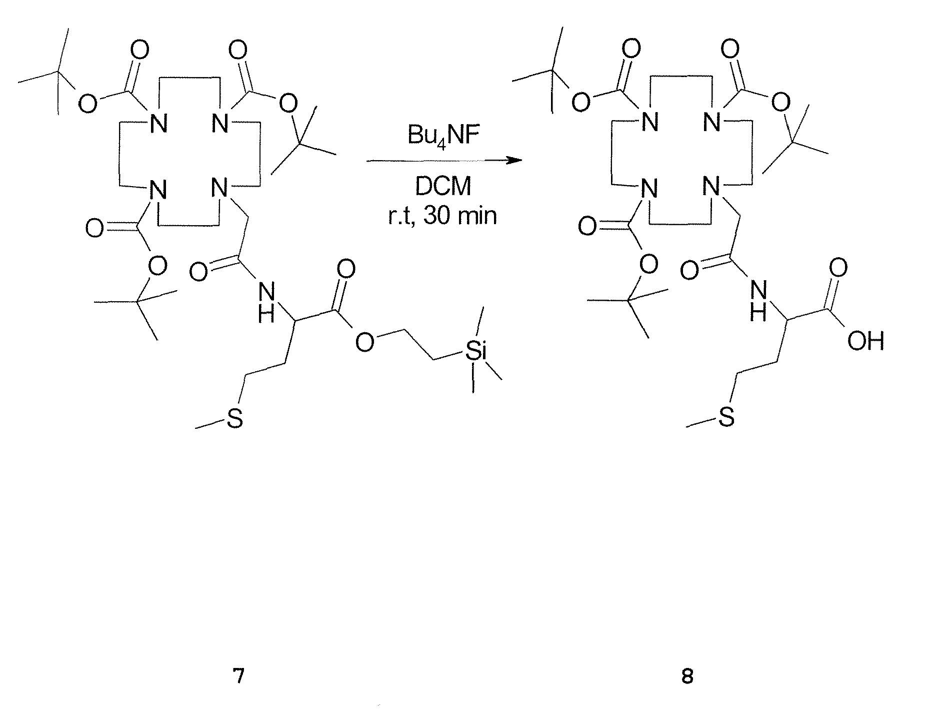

0.34 g (0.5 mmol) 7 was dissolved in 5 cm³ dichloromethane, 0.39 g (1.25 mmol) tetrabutylammonium fluoride trihydrate was added and the solution was stirred at room temperature for 30 min. The reaction mixture was poured into 35 cm³ ethyl acetate and extracted with 3 x 25 cm³ distilled water. The organic layer was dried over MgSO₄ filtered and evaporated to dryness to give a yellow oil. Yield: 0.28 g > 95% (quantitative).

### Removal of the three Boc protecting groups

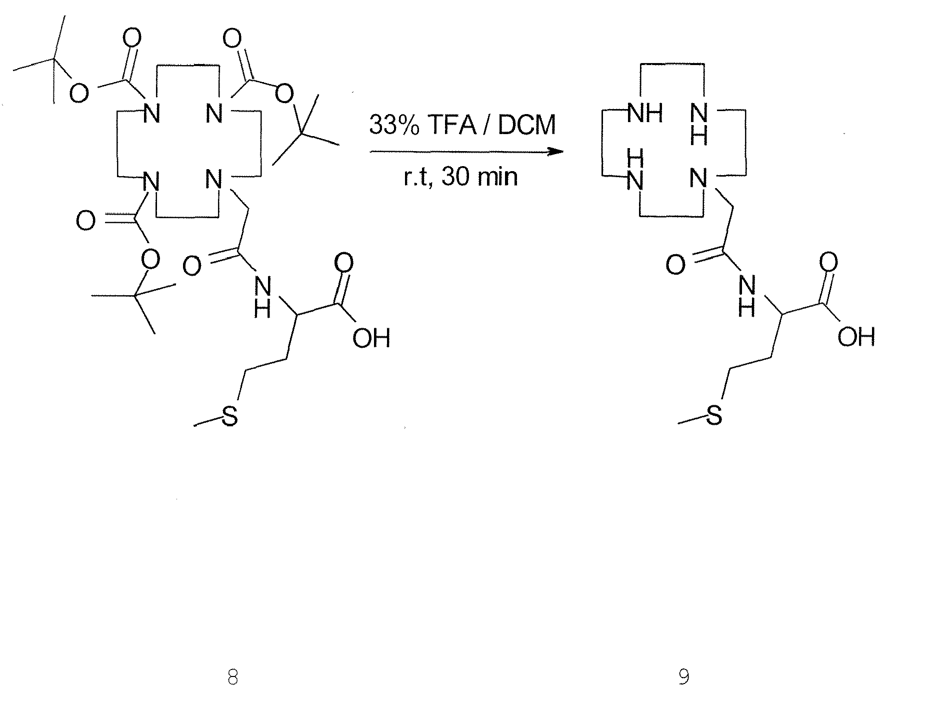

0.26 g (0.4 mmol) 8 was dissolved in 10 cm³ dichloromethane, 5 cm³ trifluoroacetic acid was added and the solution was stirred at room temperature for 30 min. The volatile components were evaporated and the sample was dried for 24 hours at high vacuum to give a brown oil. Recrystallisation from ethanol/ccHCI gave the product as an off white solid. Yield: 0.14 g, 60%.

### Complexation of rhenium with the ligand

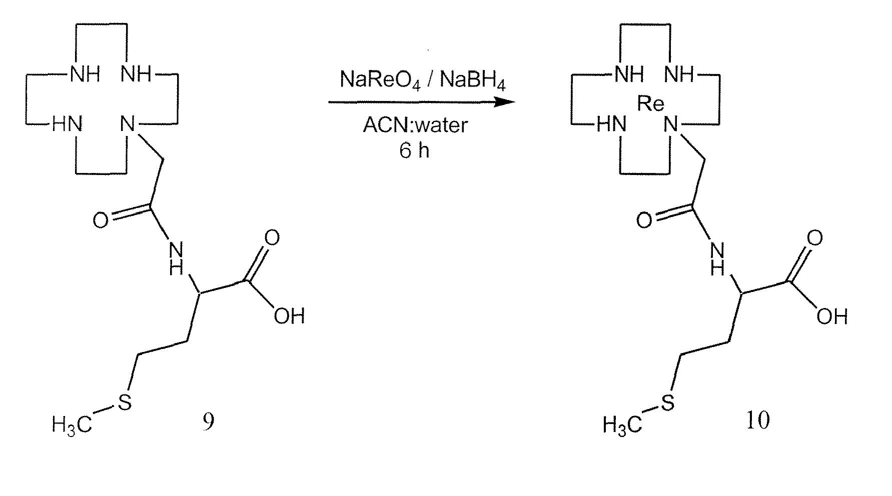

0.07 g (0.19 mmol) 9 was dissolved in 1 cm³ acetonitrile:water (1:1), 0.05 g (0.18 mmol) sodium perrhenate and 0.006 g (0.17 mmol) sodium borohydride was added. The solution was stirred at 70°C for 6 hours. The reaction was poured onto 15 cm³ diethyl ether and the resulting precipitate filtered off, washed thoroughly with diethyl ether and dried *in vacuo* to give the product as a pale solid. Yield: 0.140 g, 80%.

### Alternative synthesis of ligands

### Synthesis of D,L-Methionine-ethyl ester

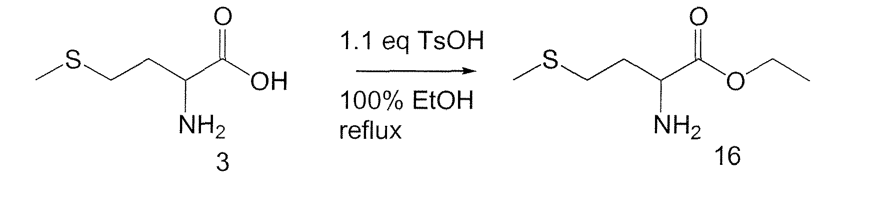

D,L-Methionine (3, 1.49 g, 10 mmol) was suspended in 100 mL 100% ethanol, toluene-p-sulphonic acid (1.89 g, 11 mmol) was added, and the solution was refluxed for 24 hours. The volatile components were evaporated at reduced pressure, and the residue triturated with cold diethyl ether. The product (16) separates out as a white, crystalline solid. The crystals were filtered out, washed on the filter with 3*30 mL diethyl ether, and dried in vacuo. Yield: 1.4g, >95%

### D,L-Methionine-ethyl ester chloroamide

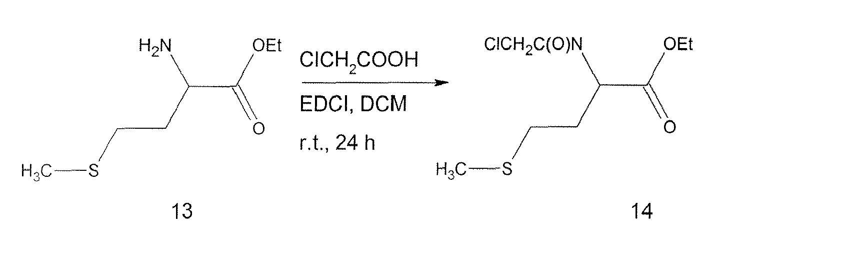

D,L-Methionine-ethyl ester (0.18 g, 1 mmol) 13 was dissolved in DCM (10 cm³), chloroacetic acid (0.18 g, 2 mmol) and EDCI (0.38 g, 2 mmol) were added, and the solution was stirred at room temperature for 24 hours. DCM (30) and water (40 cm³) were added, the phases were separated, and the aqueous phase was extracted with DCM (2*25 cm³). The combined organic phases were washed with water (30 cm³), dilute acid (30 cm³, 1 M HCI), dilute base (30 cm³, 1 M NaOH), dried over MgSO₄, filtered and evaporated to dryness to yield 14 as a colourless solid. Yield 0.23 g, >95%.

### Alkylation of tri-Boc-cyclen with D,L-Methionine ethyl ester chloroamide

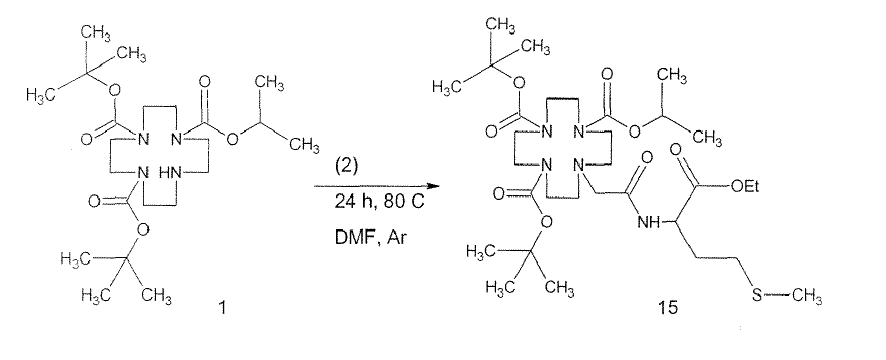

Tri-Boc-cyclen 1 (0.23 g, 0.5 mmol) was dissolved in DMF (8 cm³), K₂CO₃ (2 g), NaI (1 g) and DL-Methionine ethyl ester chloroamide (0.26 g, 1 mmol) were added and the mixture was stirred at 80 °C under argon for 24 hours. The DMF was removed *in vacuo,* the residue was suspended in DCM (50 cm³), water was added (50 cm³), the phases were separated, and the aqueous phase was extracted with DCM (3*30 cm³). The combined organic phases were washed with water (2*30 cm³), dried over MgSO₄, filtered and concentrated to ~2 cm³. The solution was applied onto a silica column and chromatographed with EtAc : Hexane (1:1). Evaporation of the solvents gave 15 as a colourless solid. Yield 0.12g, 35%.

### Hydrolysis of ester group

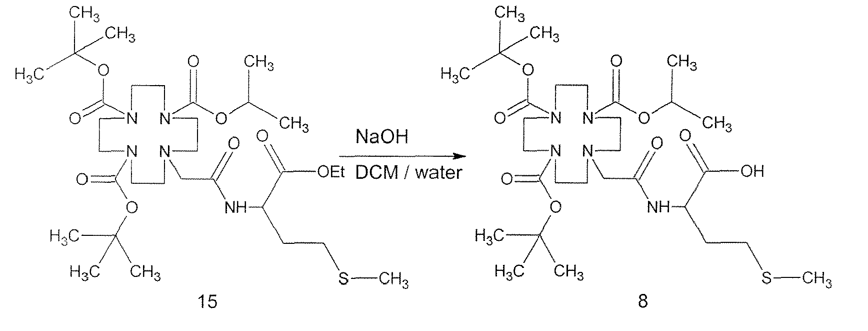

Compound 15 (0.12g) was dissolved in DCM (10 cm³), NaOH (1 M, 10 cm³) was added, and the solution was stirred vigorously at room temperature overnight. The pH was adjusted to 2 with 1:1 HC1, dichloromethane (30 cm³) was added to the mixture, the phases were separated, and the water phase was extracted with DCM (2*25 cm³). The combined organic phases were washed with water (2*30 cm³), dried over MgSO₄, filtered, and evaporated to dryness to give 8 as a white solid. Yield: 0.11g, >95%

### Removal of the Boc protecting groups

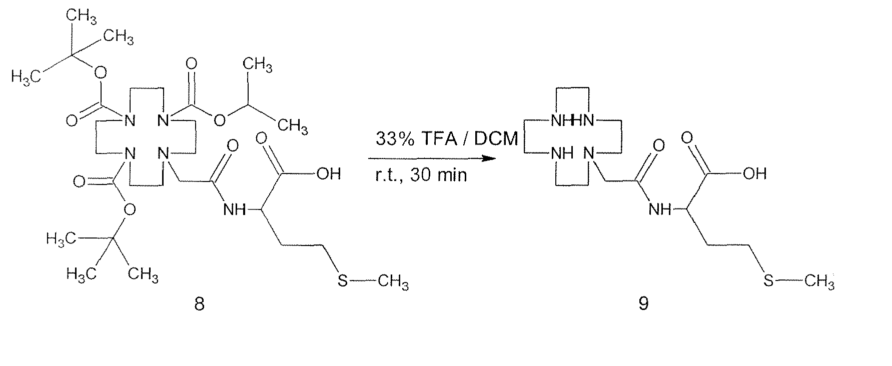

Compound 8 (0.26g, 0.4 mmol) was dissolved in dichloromethane (10 cm³), trifluoroacetic acid (5 cm³) was added and the solution was stirred at room temperature for 30 min. The volatile components were evaporated and the sample was dried for 24 hours at high vacuum. Recrystallisation from ethanol/ccHCl gave 9 as an off-white solid. Yield 0.26g, 50%.

### Esterification of 2-Carboxy benzaldehyde

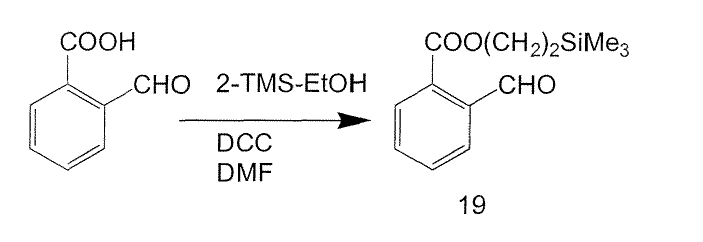

2-Carboxy benzaldehyde (1.50 g, 10 mmol) and 2-trimethylsilylethanol (1.18 g, 10 mmol) were dissolved in 10 mL dimethylformamide. Dicyclohexyl carbodiimide (2.06, 10 mmol) was added and the solution was stirred at room temperature for 12 h. The solution was diluted with 30 mL petroleum ether and 25 mL ethyl acetate and the white precipitate was filtered out. 30 mL water was added, the phases were separated and the aqueous phase was extracted with 2*30 mL ethyl acetate. The combined organic phases were washed with dilute base (30 mL 0.1 M NaOH) and water (30 mL), dried over MgSO₄, filtered, and evaporated to dryness. Compound 19 was obtained in as a white solid. Yield 1.98g, 79%.

### Synthesis of protected porphyrin ring

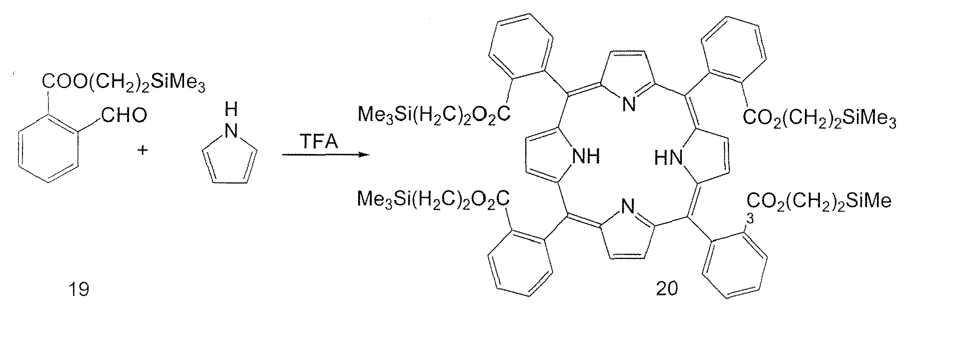

Compound 19 (0.25 g, 1 mmol) was dissolved in 250 mL dichloromethane, 0.258 mL pyrrole was added and the solution was flushed with argon for 10 minutes. The reaction was initiated with 0.288 mL trifluoroacetic acid. The reaction was conducted in the dark. After stirring at room temperature for 1.5 hours the mixture was filtered through a pad of silica and the filtrate was washed with 20-20 mL dichloromethane until the washings were colourless. The solution was concentrated and applied onto a silica column. Elution with hexanes:dichloromethane afforded the porphyrin 20. Yield 0.06g, 5%.

### Deprotection of porphyrin ring

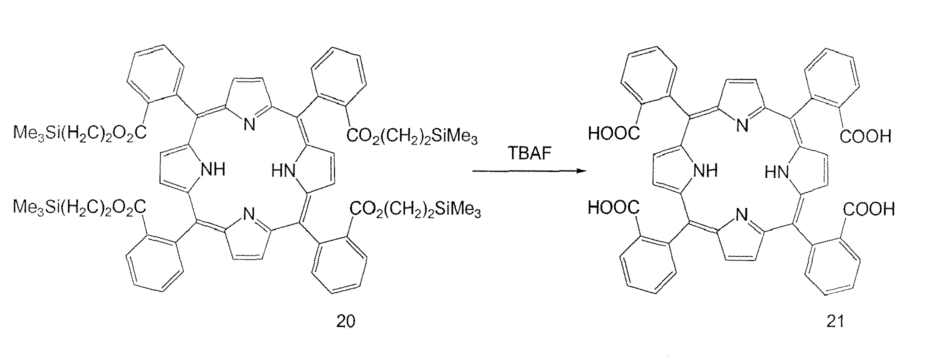

Compound 20 (0.05 g, 0.04 mmol) was dissolved in 30 mL ethyl acetate and tetrabutylammonium fluoride trihydrate (0.06 g, 0.16 mmol) was added. The solution was stirred at room temperature for 0.5 hour. The solvent was evaporated and the residue redissolved in 50 mL dichloromethane. The sample was washed with 3*25 mL water, dried over MgSO4, filtered, and evaporated to dryness. Compound 21 was obtained in quantitative yield. Yield 0.04g, >98%.

### Complexation of technetium with the ligand 9

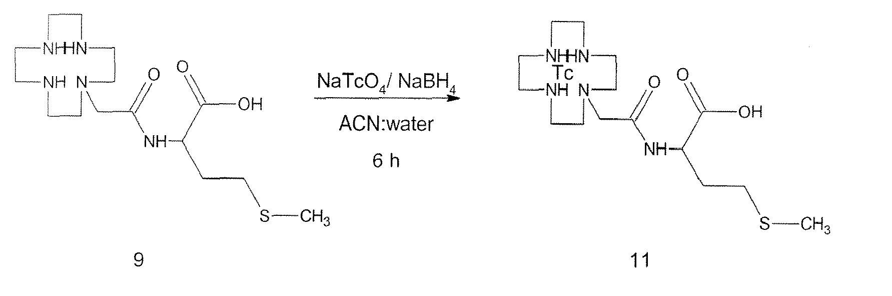

The above procedure for complexation of rhenium was carried out using sodium pertechnetate instead of sodium perrhenate to give the technetium complex 11.

### Multivalent aptamer complexes

### Materials and Methods:

**Materials:** 1-(3-dimethylaminopropyl)-3-ethylcarbodiimide hydrochloride (EDCI, coupling agent, facilitator), 1,4,7,10-tetraazacyclododecane-1,4,7,10-tetraacetic acid (DOTA) or 1,4,8,11-tetraazacyclotetradecane-1,4,8,11-tetraacetic acid tetrahydrochloride tetrahydrate (scaffold), S-acetylmercaptoacetyltriglycine (MAG3), Sodium borohydrate, (reducing agent), perhenate, ^{99m} Tc-pertechnate, Rhenium-188 dimethyl ormamide, DNAse/RTSAse free water. All materials purchased from Sigma Aldrich unless otherwise stated.

**Methods:** The reaction is prepared by creating a mixture with the following ratio: 4: 4:1-aptamer: EDCI: Cyclen. All mixtures were made up with water unless otherwise stated. The ratio is carefully calculated and mixed. The reaction is left overnight at room temperature. Next day check on an acrylamide non-denaturing gel (15%). The band of interest is excised (~40Kda) and purified either by HPLC using a C18 column or by ethanol precipitation.

The initial 4-arm complex (complex 1) is ready to be further functionalized by the coupling of MAG3 molecules to the free amino modified aptamer. At this stage the amount of aptamer bound to the cyclen can be measured by measuring the OD (units of absorbance at 260). This measurement presents the exact concentration to enable the correct calculation for the ratio to coupling MAG3 to the four arms.

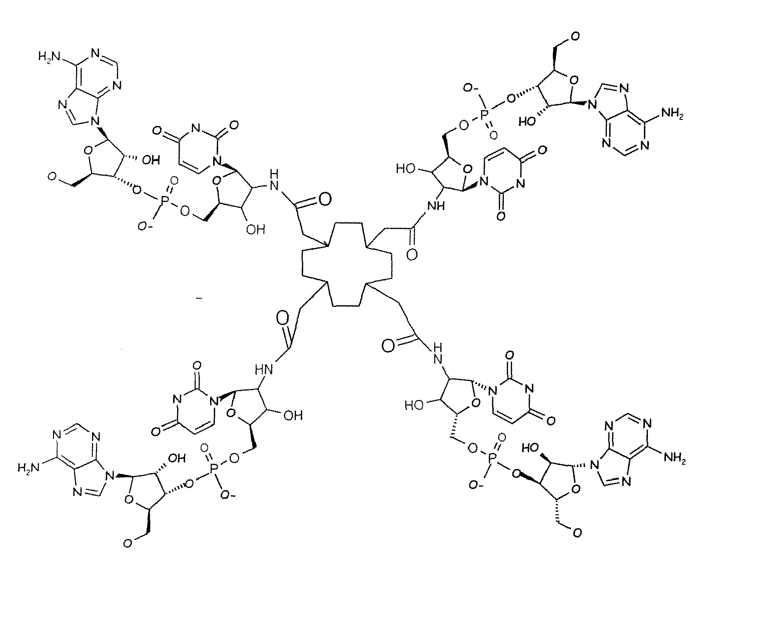

### Complex 1 - Multivalent complex using DOTA as scaffold

MAG 3 stock mix is made up as 100 times concentrated solution with 1ml of dimethylformamide 100% (DMF) (100x/per DNA concentration). A fresh EDCI stock solution is made up just before use (3x as DNA). Add this stock to the aptamer at 1:1:1 volume ration. Leave at room temperature for overnight incubation.

After incubation, at this stage, if necessary, the complex can be further purified in a NAP-10 desalting column to wash majority of salts and freeze-dried and stored until further use.

Complex 2 is similar to complex 1 but coupled MAG3 (chelator).

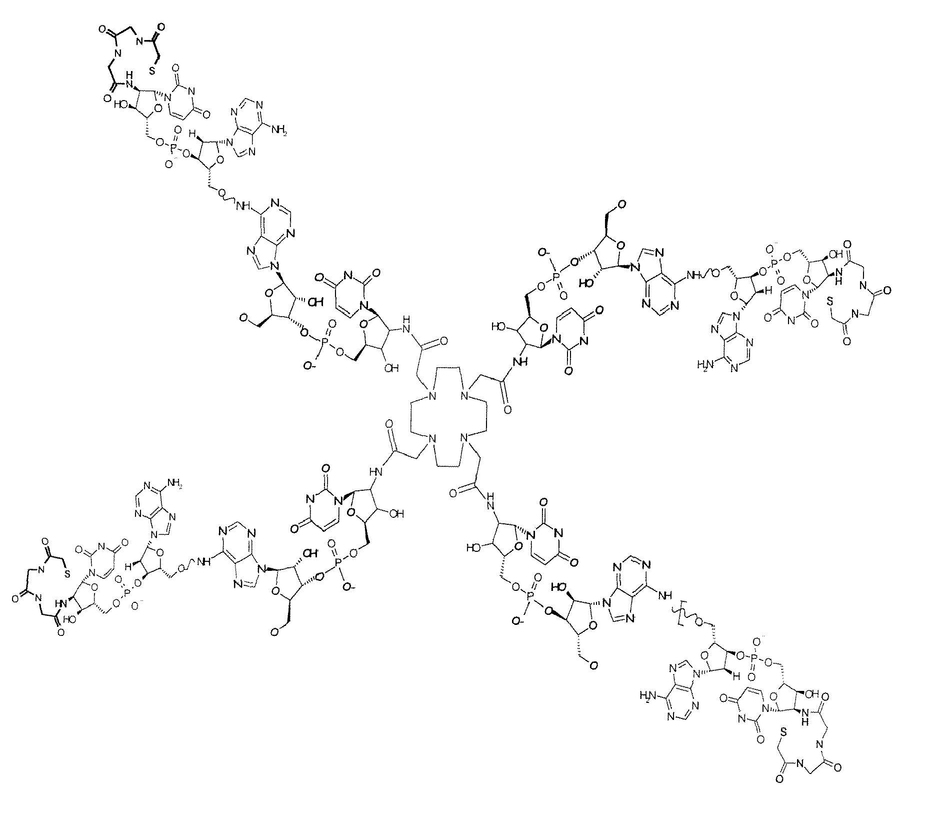

### Complex 2 - Multivalent aptamer construct with chelators

The complex functionalization is finalized by the addition of the appropriate isotope depending on the final use. ^{99m}Tc-pertechnaate and Rhenium-188 Perrhenate were used. Stock solutions of these two non-radioactive isotopes were made up to the same concentration of the stock solution of MAG3. A 1:1:1 ratio mix of the three stocks was mixed and left to incubate for 1 hour at 80°C. The final complex was then ready to be used.

### M = isotope of choice

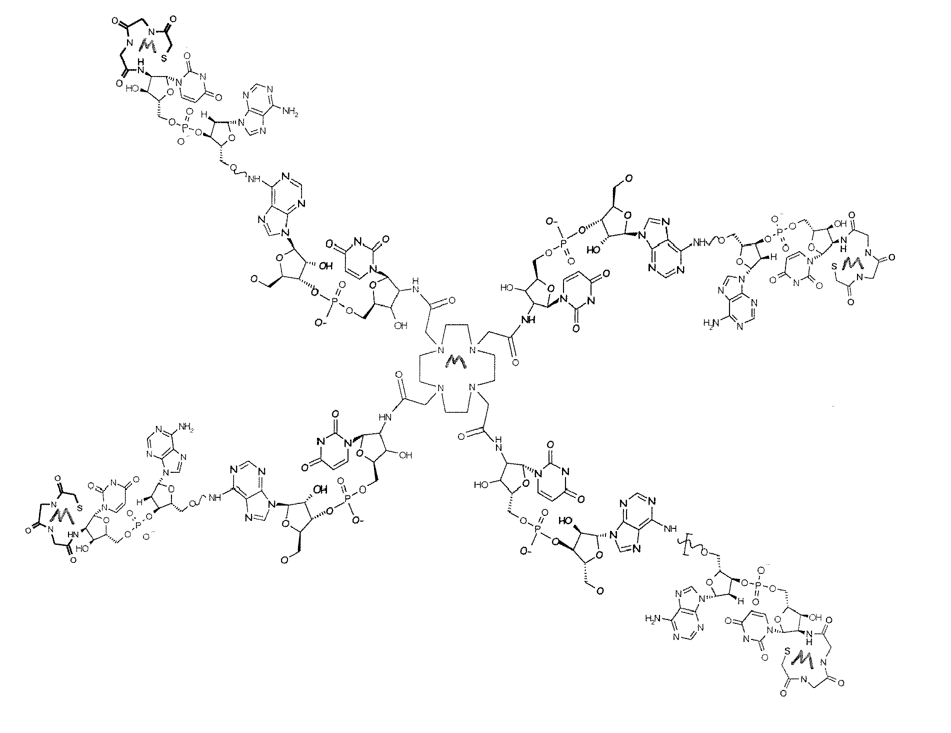

### Complex 3 - final multivalent molecule

### B: Generation, selection and identification of aptamers

### Aptamer labelling

### EXPERIMENTAL PROTOCOLS

Detailed protocols used for the identification of aptamers against MUC1 peptides. These protocols are standard protocols from either commercially available kits or previously described procedures and we make no claim as to the design of novel and unique protocols.

### 1) SELEX

### 1.1 Amplification of the library

- Reagents:: 100 µl of Library (40 µM)
100 µl of primer 1 (40 µM) (note: primers were calculated in order to obtain a single copy of every oligonucleotide present in the library)
100 µl of primer 2 (1mM)
40 µl of PBS 10x solution
40 µl of dNTPs (25 mM of each nucleotide)
18 µl of Mg2CL (15 mM)
2 µl of Taq Polymerase (native enzyme)

All reagents were mixed in a PCR thin walled tube and PCR protocol was started in the thermocycler.

### 1.1.PCR Protocol

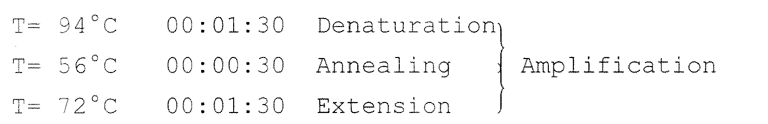

Repeat 99x (1,2,3)
T= 72°C 00:00:30 Final extension
Hold at 4°C

### 1.2 Affinity Chromatography

The column was prepared according to manufacturer's specifications. The column used is retailed by Pharmacia Biotech under the name of HiTrap (NHS-activated) Column. The column has a special activated gel matrix that will covalently couple with ligands containing primary amino groups. The peptide was loaded in the column and the concentration of bound peptide was calculated with reference to its before and after UV spectra. The column was then washed with start buffer (0.2M NaHCO₃, 0.5M NaCl, pH 8.3) to eliminate any residual unbound peptide and other unwanted residues. All protocols as per manufacturer's directions.

When the column was ready to be used the library of oligonucleotides was inserted into the column. If 1cm³ of oligonucleotide solution was used, the column was filled and left at 4 hours at 4°C or 30 min to 1 hour at room temperature. If more than 1 cm³ of oligonucleotide solution was used then a syringe was attached to each end of the column and the solution syringed back and forwards for 30 minutes at room temperature. (Note that flow rate of 1 drop/sec should be observed)

The column was then washed with 5 column volumes of starting buffer to eliminate any unbound or non-specifically, weakly bound aptamers.

The column was then eluted using 3M sodium thiocyanate solution. The eluted sample was posteriorly desalted using a NAP-10 column also by Pharmacia Biotech, using deionized and distilled water as a final buffer of a final volume of 1.5cm³.

The sample was directly collected in a 1.5 cm³ eppendorf tube and freeze-dried.

The freeze-dried sample was then mixed with the PCR reagents and put to cycle for 99x in the thermocycler.

### 1.2.1 PCR Protocol

The selected aptamers after each round were amplified by PCR. A typical PCR reaction is made up according to manufacturer instructions for optimum efficiency
- Reagents:: sample obtained from step 1.2
100 µl of primer 1 (40 µM)
100 µl of primer 2 (1 mM)
40 µl of PBS 10x solution (as provided by manufacturer)
40 µl of dNTPs (25 mM of each nucleotide)
18 µl of Mg2CL (15 mM)
2 µl of Taq Polymerase (native enzyme)
Note: Primers are used in 1/1000 of the concentrated product.

The following protocol is followed for each of the 10 rounds:

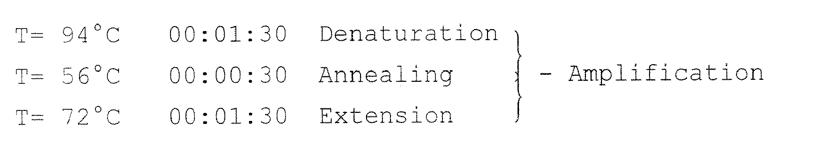

Repeat 99x (1,2,3)
T=72°C 00:00:30 Final extension
Hold 4°C

Aptamers were checked on a standard 2% Agarose gel stained with ethidium bromide.

### 2) Cloning (Using Topo TA Cloning kit by Invitrogen Life Sciences)

The table below describes the set up of the TOPO Cloning reaction with a final volume of 6 µl for eventual transformation into their chemically competent One Shot E. coli.
Reagent Chemically competent E. coli
Fresh PCR product 0.5 to 4 µl
Salt solution 1 µl
Sterile Water Add to a total volume of 5 µl
TOPO vector 1 µl
Final Volume 6 µl

The cloning reaction: The reagents were mixed and incubated for 5 minutes at room temperature (22-23°C). The reaction was placed on ice and the One Shot chemical transformation was performed as per manufacturer's directions.

One Shot chemical transformation: 2 µl of the TOPO reaction was added into a vial of One shot chemically competent E. coli and mixed gently. (Mixing must not be performed by pipetting up and down.)

The mixture was incubated on ice for 5 to 30 minutes. The cells were heat-shocked for 30 seconds at 42°C without shaking, and then the tubes were immediately transferred to ice. 250 µl of room temperature SOC medium was added. The tube was capped horizontally (200 rpm) at 37°C for 1 hour.

10-50 µl from each transformation was spread on a prewarmed selective plate and incubated overnight at 37°C. 10 white colonies were picked and then positive clones were analysed.

### 2.1 Analyses of Positive Clones

PCR may be used to analyze positive transformants.

A PCR cocktail was prepared which consisted of PCR buffer, dNTPs, primers and Taq polymerase. A 20 µl reaction volume was used, multiplied by the number of colonies to be analysed. 10 colonies were picked and resuspended individually in 20 µl of the PCR cocktail. The colony was patched onto a separate plate to preserve for future use.

The reaction was incubated for 10 min at 94°C to lyse cells and inactivate nucleases. Amplification for 20 to 30 cycles (94° 1 min, 55°C for 1 minute, and 72°C for one minute) was performed. A final extension was performed by incubating at 72°C for 10min. The mixture was then held at 4° C.

Visualisation was performed by standard agarose gel electrophoresis. A bright band indicated cloning of the insert.

### 2.3 Recipes for LB media and others

LB (Luria Bertani) Medium and Plates:

### Composition

1% Tryptone 10g
0.5% Yeast Extract 5g
1.0% NaCl 10g
1000ml H₂O 18Ω

The reagents were dissolved in 950cm³ deionised water. The pH of the solution was adjusted with NaOH and the volume was brought up to 1 liter. The solution was autoclaved on a liquid cycle for 20 min at 15 psi. The solution was allowed to cool to 55°C and an antibiotic was added (50 µg/cm³ of ampicillin). The solution was stored at room temperature or at 4°C.

### LB agar plates

LB medium was prepared as before, but 15g/L of agar was added before autoclaving was performed. The solution was autoclaved for 20 min at 15psi. After autoclaving the solution was allowed to cool to 55°C and an antibiotic was added. The solution was poured into 10cm plates. After the solution hardened, the plates were inverted and stored at 4°C, in the dark.

### X-Gal stock solution

A 40mg/cm³ stock solution was prepared by dissolving 400mg X-Gal in 10 cm³ DMF (dimethylformamide). The solution was protected from light by storing in a brown bottle at -20°C. To add to previously made agar plates, the plate was warmed to 37°C. 40 µl of the 40mg/cm³ stock solution was pipetted onto the plate, spread evenly and allowed to dry for 15 minutes. Protect plates from light.

### IPTG (Isopropyl-beta-D-thiogalactopyranoside)

A 100mM stock solution was prepared by dissolving 238mg of IPTG in 10cm³ deionised water. The solution was filter-sterilized and stored in 1cm³ aliquots at -20°C. 40 µl of the IPTG stock solution was added onto the center of a plate and spread evenly with a sterile spreader. The solution was allowed to diffuse into the plate by incubating at 37°C for 20-30min.

### 3) Isolation of plasmid from the bacterial colony

1.5cm³ of the bacterial culture was spun down in a microcentrifuge vial for 30 seconds. The supernatant was discarded and 0.75cm³ of culture was added to the vial containing the pellet and the mixture was spun down for an additional 30 seconds. The supernatant was removed and discarded completely.

The pellet was then resuspended in 50 µL Pre-Lysis Buffer. The solution was mixed by vortexing until the pellet was completely resuspended.

100 µL Alkaline lysis solution was added directly into the cell suspension and the solution was mixed thoroughly.

75 µL of neutralising solution was added and the solution was vortexed, then spun in a microcentrifuge for 2 min. The supernatant was carefully transferred to a spin filter.

250 µL binding buffer was added to the spin filter, and centrifuged for another minute.

Ethanol was added to washing solution as per manufacturer's instructions before use. 750 µL of the washing solution was added to the spin filter and centrifuged for 1 min. The liquid was poured off and another 250 µL of the washing solution was added. The solution was centrifuged for another minute.

The filter was spun for another 2 minutes to dry the pellet. The spin filter was transferred to a new vial. 50 µL of nuclease free water was added and spun for another minute. The spin filter was discarded and the sample was stored at -20°C. The DNA solution was ready to be used.

### SEQUENCE LISTING FREE TEXT

| SEQ ID NO. | Free Text <223> |
|---|---|
| 1 | Aptamer i |
| 2 | Aptamer ii |
| 3 | Aptamer iii |
| 4 | Aptamer iv |
| 5 | Aptamer v |
| 6 | Aptamer vi |
| 7 | Aptamer vii |
| 8 | Aptamer viii |
| 9 | Aptamer ix |
| 10 | Aptamer x |
| 11 | Aptamer xi |
| 12 | Aptamer xii |
| 13 | Aptamer xiii |
| 14 | Aptamer xiv |
| 15 | Aptamer xv |
| 16 | Aptamer xvi |
| 17 | Aptamer xvii |
| 18 | Aptamer xviii |
| 19 | Aptamer xix |
| 20 | Aptamer xx |
| 21 | Aptamer xxi |
| 22 | Aptamer xxii |

## Claims

1. A compound having the structure: 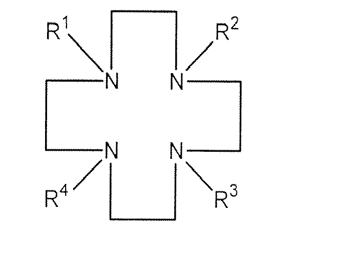 wherein
R¹, R², R³ are each independently:
hydrogen, R⁴, R⁵, R⁵-carbonyl, R⁵-sulphonyl;
R⁴ comprises an amino acid moiety;
R⁵ is:
alkyl, alkenyl, alkynyl, cycloalkyl, aryl, heteroaryl, heterocyclyl, wherein each of these groups is optionally substituted by one or more of:
alkyl, amino, amino-carbonyl, amino-sulphonyl, halo, cyano, hydroxy, nitro, trifluoromethyl, alkoxy, alkoxycarbonyl, aryl and alkylthio;
and salts and solvates thereof.

2. A compound according to claim 1, wherein R⁴ comprises a lysine, cysteine, glycine, threonine, serine, arginine or methionine moiety.

3. A compound according to claim 1 or 2, wherein R¹, R² and R³ are hydrogen.

4. A compound according to claim 1 or 2, wherein at least one of R¹, R² and R³ is alkyl.

5. A compound according to any preceding claim, wherein R⁴ comprises a carboxyl or amino group.

6. A compound according to any preceding claim, wherein R⁴ comprises a nitrogen, oxygen or sulphur atom.

7. A compound according to claim 1, wherein R⁴ has the structure:

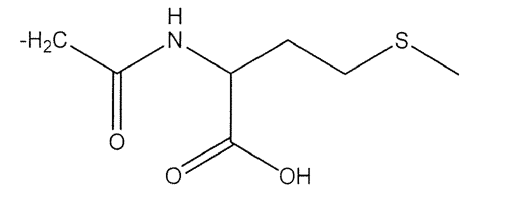

8. A compound according to claim 1, having the structure:

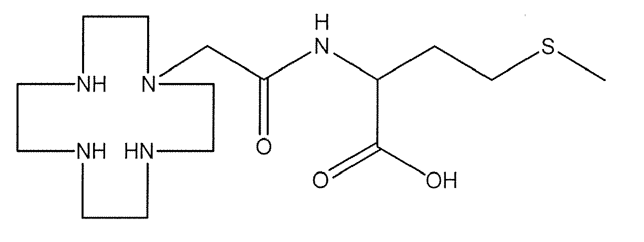

9. A compound according to any preceding claim, further containing a complexing metal (M).

10. A compound according to claim 9, having the structure: 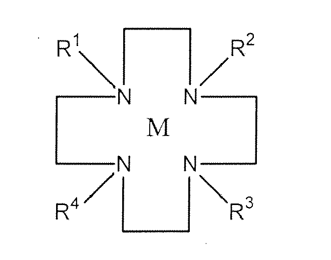 wherein R¹, R², R³, R⁴ and M are as defined in any preceding claim; and salts and solvates thereof.

11. A compound according to claim 9 or 10, wherein M is rhenium, technitium or yttrium.

12. A compound according to any of claims 9 to 11, wherein M is a radioisotope.

13. A pharmaceutical composition comprising a compound as defined in any of the preceding claims and a pharmaceutically acceptable carrier.

14. A method of imaging a disease or condition comprising the step of administering a compound or pharmaceutical composition as defined in any preceding claim.

15. A method according to claim 14, wherein the disease or condition is associated with the over-production of MUC1.

16. A method according to claim 14 or 15, wherein the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

17. A method according to any of claims 14 to 16, wherein MUC1 bearing and/or expressing cells are irradiated by the compound, aptamer or composition.

18. A compound or composition according to any of claims 1 to 13, for use in therapy.

19. A compound or composition according to any of claims 1 to 13 or 18, for use in the treatment prevention, diagnosis, detection and/or imaging of a condition or disease.

20. A compound or composition according to claim 19, wherein the condition or disease is associated with the over-production of MUC1.

21. A compound or composition according to claim 19 or 20, wherein the condition or disease is selected from cancer,cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

22. Use of a compound as defined in any of claims 1 to 12, for: the manufacture of a medicament for the prevention and/or treatment of a disease or condition associated with the over-production of MUC1; the manufacture of a product for the diagnosis of a disease or condition associated with the over-production of MUC1; the manufacture of a product for the detection of a disease or condition associated with the over-production of MUC1, or the manufacture of a product for the imaging of a disease or condition associated with the over-production of MUC1.

23. Use of a compound according to claim 22, wherein the disease or condition is selected from cancer, cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer.

24. A kit for the prevention, treatment, diagnosis, detection and/or imaging of a disease or condition comprising a compound or composition according to any of claims 1 to 13.

25. A kit for the prevention, treatment, diagnosis, detection and/or imaging of a disease or condition associated with the over-production of MUC1 comprising a compound or composition according to any of claims 1 to 13.

26. A kit for the prevention, treatment, diagnosis, detection and/or imaging of a disease or condition selected from cancer,cystic fibrosis, asthma, chronic obstructive lung disease, non-malignant inflammatory epithelial disease, adenocarcinoma, breast cancer, colon cancer, pancreatic cancer, lung adenocarcinoma, non-small cell lung cancer, ovarian cancer, stomach cancer, prostate cancer, endometrium cancer and colorectal cancer, comprising a compound or composition according to any of claims 1 to 13.

27. A method of preparing a compound as defined in any of claims 1 to 8 or salts and solvates thereof; comprising the step of reacting a cyclen compound with an amino acid moiety.

28. A method according to claim 27 having the following synthetic route:

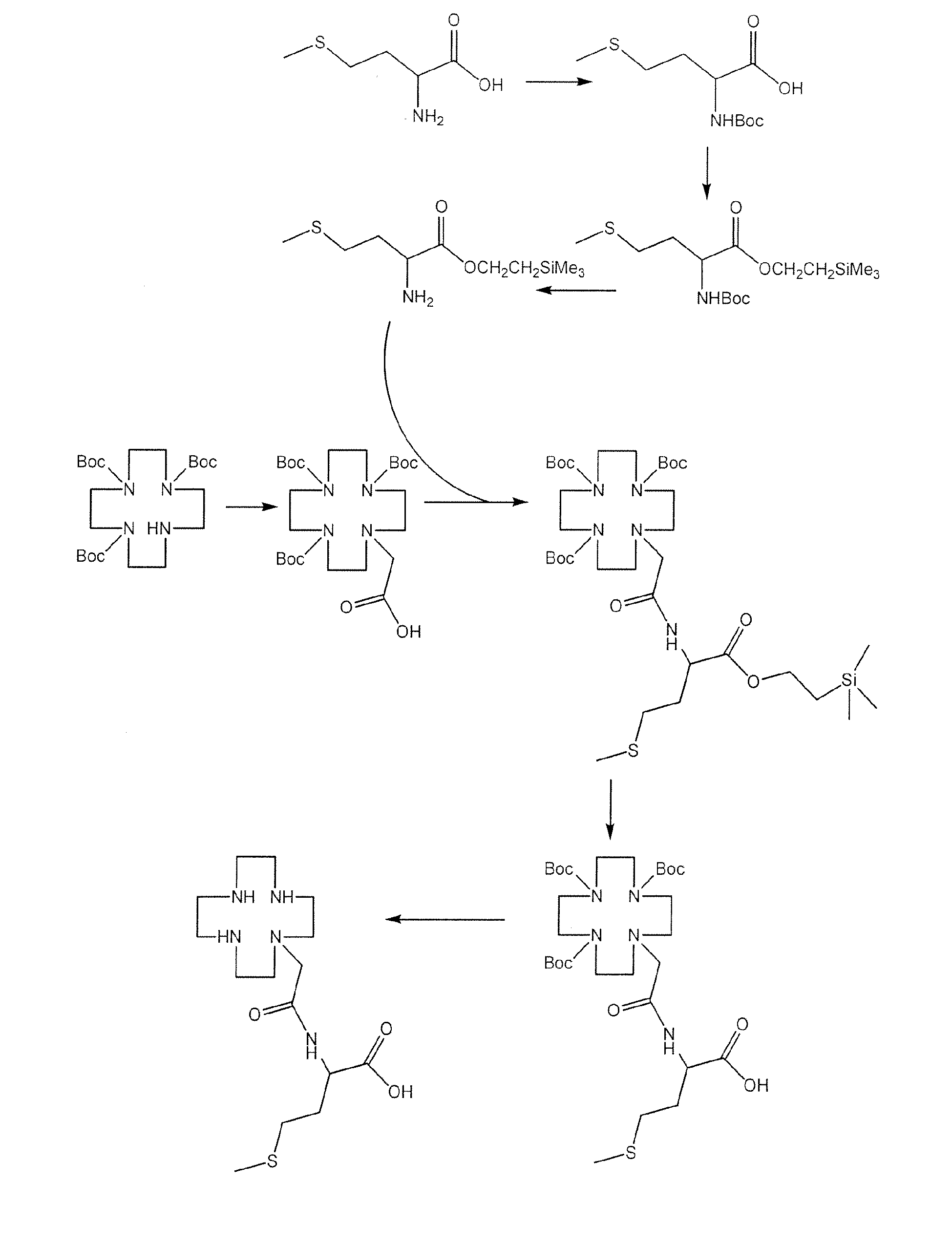

29. A method according to claim 27 having the following synthetic route:

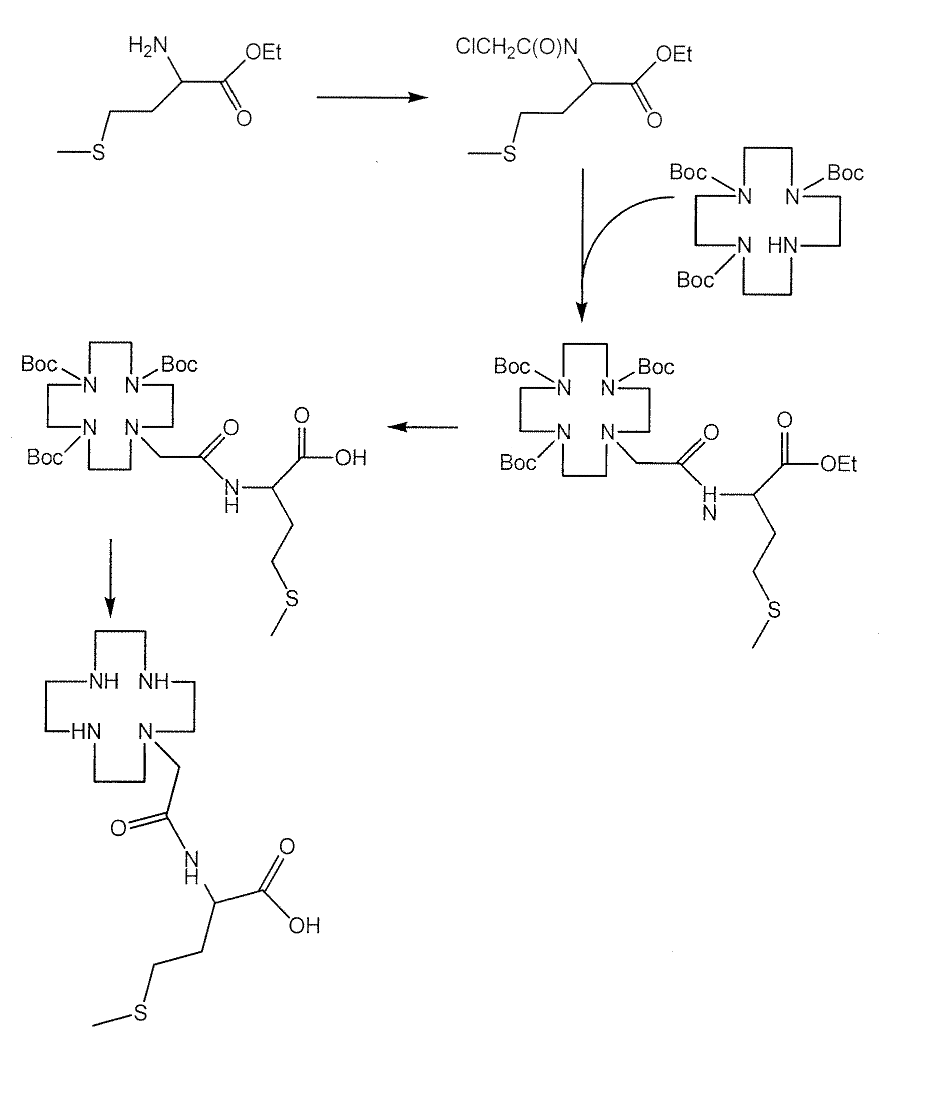

30. A method of complexing a compound with a complexing metal (M) comprising the step of admixing a compound as defined in any of claims 1 to 8 or salts and solvates thereof; with a source of M.

31. A method according to claim 30, comprising the step of reacting the compound with NaMO₄/NaBH₄.

32. A method according to claim 30 or 31, wherein M is selected from rhenium, technitium and yttrium.

33. A method according to any of claims 30 to 32, wherein M is a radioisotope.


SEQUENCE LISTING

<110> The Open University
Missailidis, Sotiris
Bruce, James I.
Ferreira, Catia S. M.
Borbas, Katalin E.

<120> Detection, monitoring and treatment of cancer

<130> RA/GDP/P302887WO

<150> GB0305422.8
<151> 2003-03-10

<160> 22

<170> PatentIn version 3.1

<210> 1
<211> 25
<212> DNA
<213> Artificial Sequence

<220>
<223> Aptamer i
<400> 1
cgaatgggcc cgtcctcgct gtaag 25

<210> 2
<211> 25
<212> DNA
<213> Artificial Sequence

<220>

<223>  Aptamer ii

<400>  2
gcaacagggt atccaaagga tcaaa                                          25

<210>  3

<211>  25

<212>  DNA

<213>  Artificial Sequence

<220>

<223>  Aptamer iii

<400>  3
gttcgacagg aggctcacaa caggc                                          25

<210>  4

<211>  25

<212>  DNA

<213>  Artificial Sequence

<220>

<223>  Aptamer iv

<400>  4
tgttggtcag gcggcggctc tacat                                          25

<210>  5

<211>  25

<212>  DNA

<213>  Artificial Sequence

<220>

<223>  Aptamer v

<220>

<221>  misc_feature

<222>  (22)..(25)

<223>  n may be any nucleotide

<400>  5
ctctgttctt atttgcgagt tnnnn                                          25

<210>  6
<211>  25
<212>  DNA
<213>  Artificial Sequence

<220>
<223>  Aptamer vi
<220>
<221>  misc_feature
<222>  (1)..(1)
<223>  n may be any nucleotide

<220>
<221>  misc_feature
<222>  (23)..(25)
<223>  n may be any nucleotide

<400>  6
nctctgttct tatttgcgag ttnnn                                          25

<210>  7
<211>  25
<212>  DNA
<213>  Artificial Sequence

<220>
<223>  Aptamer vii
<220>
<221>  misc_feature
<222>  (1)..(2)
<223>  n may be any nucleotide

<220>
<221>  misc_feature

<222>  (24)..(25)
<223>  n may be any nucleotide

<400>  7
nnctctgttc ttatttgcga gttnn                                          25

<210>  8
<211>  25
<212>  DNA
<213>  Artificial Sequence

<220>
<223>  Aptamer viii
<220>
<221>  misc_feature
<222>  (1)..(3)
<223>  n may be any nucleotide

<220>
<221>  misc_feature
<222>  (25)..(25)
<223>  n may be any nucleotide

<400>  8
nnnctctgtt cttatttgcg agttn                                          25

<210>  9
<211>  25
<212>  DNA
<213>  Artificial Sequence

<220>
<223>  Aptamer ix
<220>
<221>  misc_feature
<222>  (1)..(4)

<223>  n may be any nucleotide

<400>  9
nnnnctctgt tcttatttgc gagtt                                          25

<210>  10
<211>  21
<212>  DNA
<213>  Artificial Sequence

<220>
<223>  Aptamer x
<400>  10
ctctgttctt atttgcgagt t                                              21

<210>  11
<211>  25
<212>  DNA
<213>  Artificial Sequence

<220>
<223>  Aptamer xi
<400>  11
ccctctgttc ttatttgcga gttca                                          25

<210>  12
<211>  25
<212>  DNA
<213>  Artificial Sequence

<220>
<223>  Aptamer xii
<400>  12
ctctgttctt atttgcgagt tggtg                                          25

<210>  13
<211>  25
<212>  DNA

<213>  Artificial Sequence

<220>

<223>  Aptamer xiii

<400>  13
ccctctgttc ttatttgcga gttca                                          25

<210>  14

<211>  25

<212>  DNA

<213>  Artificial Sequence

<220>

<223>  Aptamer xiv

<400>  14
taagaacagg gcgtcgtgtt acgag                                          25

<210>  15

<211>  25

<212>  DNA

<213>  Artificial Sequence

<220>

<223>  Aptamer xv

<400>  15
gtggcttact gcgaggacgg gccca                                          25

<210>  16

<211>  25

<212>  DNA

<213>  Artificial Sequence

<220>

<223>  Aptamer xvi

<400>  16
gcagttgatc ctttggatac cctgg                                          25

<210>  17

<211>  25

<212>  DNA

<213>  Artificial Sequence

<220>

<223>  Aptamer xvii

<400>  17
aaccctatcc acttttcggc tcggg                                              25

<210>  18

<211>  25

<212>  DNA

<213>  Artificial Sequence

<220>

<223>  Aptamer xviii

<400>  18
cgatttagtc tctgtctcta ggggt                                              25

<210>  19

<211>  25

<212>  DNA

<213>  Artificial Sequence

<220>

<223>  Aptamer xix

<400>  19
cgacaggagg ctcacaacag gcaac                                              25

<210>  20

<211>  25

<212>  DNA

<213>  Artificial Sequence

<220>

<223>  Aptamer xx

<400>  20
agaacgaagc gttcgacagg aggct                                              25

<210>  21

<211>  25

<212>  DNA

<213>  Artificial Sequence

<220>

<223>  Aptamer xxi

<400>  21
agaaacactt ggtatatcgc agata                                          25

<210>  22

<211>  25

<212>  DNA

<213>  Artificial Sequence

<220>

<223>  Aptamer xxii

<400>  22
gggagacaag aataaacact caacg                                          25